(19) Europäisches Patentamt
European Patent Office
Office européen des brevets

(11) EP 1 215 282 A1

(12) EUROPEAN PATENT APPLICATION
published in accordance with Art. 158(3) EPC

(43) Date of publication:
**19.06.2002 Bulletin 2002/25**

(21) Application number: **00957006.0**

(22) Date of filing: **07.09.2000**

(51) Int Cl.[7]: **C12N 15/02**, C12P 21/08, C12N 15/12, G01N 33/53, G01N 33/577

(86) International application number:
**PCT/JP00/06100**

(87) International publication number:
**WO 01/18196 (15.03.2001 Gazette 2001/11)**

(84) Designated Contracting States:
**AT BE CH CY DE DK ES FI FR GB GR IE IT LI LU MC NL PT SE**
Designated Extension States:
**AL LT LV MK RO SI**

(30) Priority: **07.09.1999 JP 25344399**
**29.10.1999 JP 31018599**

(71) Applicant: **KYOWA MEDEX CO., LTD.**
**Tokyo 104-0042 (JP)**

(72) Inventors:
- **KOHNO, Hiroaki, Kyowa Medex Co. Ltd.**
  **Sunto-gun, Shizuoka 411-0932 (JP)**
- **HASHIMOTO, Yuriko, Kyowa Medex Co. Ltd.**
  **Sunto-gun, Shizuoka 411-0932 (JP)**
- **YOSHIZAWA, Takumi**
  **Kita-gun, Kagawa 761-0612 (JP)**

(74) Representative: **VOSSIUS & PARTNER**
**Siebertstrasse 4**
**81675 München (DE)**

# (54) METHOD OF DETECTING TRICHOTHECENE MYCOTOXINS AND REAGENT THEREOF

(57) Monoclonal antibodies having high affinity for trichothecene mycotoxins DON, NIV and T-2 are produced, and the trichothecene mycotoxins are collectively determined by using the antibodies.

EP 1 215 282 A1

## Description

### Technical Field

The present invention relates to an immunoassay for trichothecene mycotoxins in crops, foods, feeds, etc., a reagent and a reagent kit for the immunoassay, and monoclonal antibodies for use therein.

### Background Art

Mycotoxins are defined as secondary metabolites produced by fungi which have some harmful effect on humans or animals via foods or feeds, and are distinguished from bacterial toxins and the like. Mycotoxins, unlike bacterial toxins, are low molecular weight substances and include various known substances. There is considerable variation in their chemical structure and harmful effect on living organisms. Not a few of them are harmful to humans; some show a high acute toxicity in the human body, and others are recognized to have carcinogenicity or tumorigenicity at kidney and liver. Because of low molecular weight, mycotoxins are difficult to decompose or remove under ordinary conditions of food processing and cooking. Further, the high stability of mycotoxins allows them, when taken into food animals via feeds, to remain in the meat and milk products from the animals and to finally show toxicity after intake into the human body.

Mycotoxin contamination can occur by various routes, but is broadly classified into the primary contamination of crops occurring by the invasion of fungi in the process of cultivation, harvesting, storage and processing of crops, and the secondary contamination of livestock and marine products (e.g., meat, milk and eggs) occurring by feeding with contaminated feeds. The primary contamination shows local characteristics according to factors such as the kind of crops (substrates for fungi), the kind of fungi occurring in a cultivation environment, and environmental conditions (e.g., temperature, humidity and precipitation).

Trichothecene mycotoxins are representative mycotoxins contaminating major cereal crops such as barley, wheat, rye, oates and maize. Among the representative trichothecene mycotoxins are deoxynivalenol (DON), nivalenol (NIV) and T-2 toxin (T-2). The fungi mainly producing them, Fusarium graminearum, F. culmorum and F. sporotrichioides, commonly occur in soil and easily infect crops if natural conditions such as temperature, humidity and precipitation are satisfied, leading to spread of mycotoxin contamination of crops.

With the aim of preventing contamination of foods and feeds, regulations regarding the residual concentrations of trichothecene mycotoxins in foods and feeds are adopted in many countries. Accordingly, it is important to rapidly and accurately determine trichothecene mycotoxins in foods and feeds.

Previous methods for the determination of trichothecene mycotoxins include thin layer chromatography, high performance liquid chromatography, gas chromatography, mass spectrometry and bioassay using animals, which are carried out singly or in combination. Further, more recently proposed immunoassays came into use. Immunoassays, which are rapid and simple to execute and are excellent in specificity and sensitivity, are widely applied to various fields including determination of hormones and biological substances. Especially, application of techniques for preparing monoclonal antibodies has promoted great advances in immunoassay methods.

There exist numbers of trichothecene mycotoxin derivatives, which are also toxic. Therefore, in order to know the degree of trichothecene mycotoxin contamination, it is preferred to determine the derivatives somewhat collectively rather than individually. The subjects to be assayed include a very wide range of substances; for example, cereals such as barley, wheat, rye, oates and maize infected with trichothecene mycotoxins, meat and milk of livestock fed with the infected cereals, processed foods produced from the infected meat and milk, and processed foods produced from the infected cereals. Further, as the concentrations of mycotoxins are diluted during the processing, it is necessary to determine trichothecene mycotoxins at low concentrations. Thus, a need exists for an assay method with a high sensitivity.

On the other hand, in order to know the place and situation of contamination, it is important to determine the three major trichothecene mycotoxins, DON, NIV and T-2, individually. For establishing immunoassays capable of such determination of trichothecene mycotoxins, antibodies having a high affinity and a high specificity for the trichothecene mycotoxin to be determined, especially, monoclonal antibodies will be advantageously used.

Monoclonal antibodies for trichothecene mycotoxins are disclosed in various publications. Japanese Published Examined Patent Application No. 43358/93 discloses monoclonal antibodies specific for T-2 which are obtained by immunizing mouse with an immunogen prepared by conjugating T-2 to a carrier protein and an assay method for T-2 group using the antibodies. Dietrich, et al. obtained monoclonal antibodies for T-2 and monoclonal antibodies for 3-acetyl DON (Natural Toxins, 3:288-293, 1995). U.S. Patent No. 4879248 discloses production of antibodies by conjugating a carrier protein using the substituent at the 8-position as a linker. However, none of these publications disclose monoclonal antibodies having a high affinity and the specificity suited for the object of the present invention.

Applied and Environmental Microbiology, May 1993, 59:1264-1268 discloses polyclonal antibodies for

4,15-diacetyl NIV prepared by using an immunogen in which a carrier protein is bound to the carbon at the 3-position, but it contains no disclosure about production of monoclonal antibodies using a similar immunogen. Further, monoclonal antibodies for T-2 and DON are disclosed in Food Addit. Contam., 5:629 (1988) and J. Agric. Food Chem., 36:663 (1988).

The monoclonal antibodies according to the present invention have the following characteristics and are superior to the known monoclonal antibodies mentioned above.

KTM-249 has a remarkably high affinity, compared with the antibody of Dietrich, et al. (3E2) [Natural Toxins, 3:288 (1995)], and is highly sensitive (detection limit for T-2: ca. 0.0001 ng/ml). KTM-249 shows the same degree of reactivity with acetyl T-2, HT-2 and T-2, and thus is suitable for the object of the present invention.

The reactivities of KTM-249 with HT-2 and T-2 are at the same level (T-2 = HT-2 = 100%) and its reactivity with acetyl T-2 is 78%. On the other hand, the anti-T-2 monoclonal antibody IVI-10092 disclosed in U.S. Patent No. 4772551 showed 50% inhibition values of 0.023 for T-2, 0.094 for acetyl T-2 (reactivity: 0.023/0.094 ≒ ca. 25%, based on the 50% inhibition value for T-2 as 100%) and 1 for HT-2 (reactivity: 0.023/1 = 2.3%). This indicates that KTM-249 is capable of detecting a wide range of T-2 derivatives, that is, it is highly selective for T-2 derivatives but is unreactive with DON or NIV derivatives, while IVI-10092 is selective only for T-2.

KTM-240 shows reactivity clearly different from that of the antibody of Dietrich, et al. (5B2) [Natural Toxins, 3:288 (1995)]. For instance, the reactivity of 5B2 with diacetyl-DON is about 6 times that with 3-acetyl-DON, and its reactivity with triacetyl-DON is less than that with 3-acetyl-DON, whereas the reactivity of KTM-240 with diacetyl-DON is about 50 times that with 15-acetyl-DON, and its reactivity with triacetyl-DON is twice that with 15-acetyl-DON. Additionally, KTM-240 is strongly reactive with acetylated NIV, whereas 5B2 is unreactive with such compounds and thus can not be used for simultaneous detection of DON and NIV.

The anti-T-2 toxin monoclonal antibody described in Japanese Published Examined Patent Application No. 43358/93 shows the cross-reactivity with HT-2 of about 3%. On the other hand, KTM-249 shows the same level of reactivity with all of HT-2, T-2 and acetyl T-2, and thus is suitable for the determination of total T-2 related toxins, which is an object of the present invention.

The reactivity of the antibody of Chiba, et al. [Food Addit. Contam., 5:629 (1988)] with HT-2 is less than 0.5% of that with T-2, while KTM-249 of the present invention strongly reacts also with HT-2.

The antibody DON-1 of Casale, et al. [J. Agric. Food Chem., 36:663 (1988)] and KTM-240 of the present invention are clearly different in reactivity. DON-1 strongly reacts with 3-acetyl-DON, reacts well with DON and weakly reacts with NIV, but does not react with 15-acetyl-DON. KTM-240 does not react with DON or NIV, but strongly reacts with diacetyl-DON and triacetyl-NIV. KTM-240 is also reactive with 15-acetyl-DON, and its reactivity with 15-acetyl-DON is stronger than that with 3-acetyl-DON.

## Disclosure of the Invention

Antibodies to be used in an immunoassay for trichothecene mycotoxins are required to be capable of determining trichothecene mycotoxins and its numerous derivatives collectively and to have specificity sufficient for determining the three major groups (i.e., DON group, NIV group and T-2 group) individually and also a high affinity enabling the determination of antigens at low concentrations. Further, in view of the high productivity and reproducibility and the constant specificity required for the purpose of the present invention, the antibodies of the present invention are preferably monoclonal antibodies or fragments thereof.

The antibody fragments include Fab, Fab' and $F(ab)_2$.

The antibody fragments of the present invention can be obtained by subjecting an antibody produced by hybridomas to enzymatic treatment using pepsin or the like, or to reduction. The antibody fragments can also be obtained by extracting mRNA from hybridomas, inserting cDNA prepared from the mRNA into an expression vector for a procaryote or eucaryote, introducing the vector into a procaryote or eucaryote, and then expressing the desired product therein.

Trichothecene mycotoxins are low molecular weight substances which are so-called haptens and have only low immunogenicity. Therefore, for the production of an antibody for a trichothecene mycotoxin, it is necessary to change the mycotoxin into a form recognizable as an antigen by a living organism, for example, by conjugating it to a carrier substance, prior to immunization. The present inventors noted the fact that the difference of binding site in preparing a conjugate of a carrier substance and a trichothecene mycotoxin affects the affinity and specificity of the antibody produced. As trichothecene mycotoxins are hardly soluble in water, their solutions are generally prepared using organic solvents. The present inventors have found that an antibody with very high affinity can be obtained by dissolving a trichothecene mycotoxin represented by formula (I):

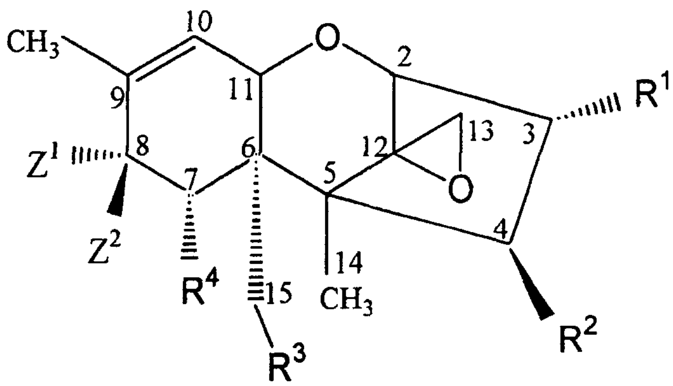

(I)

(wherein $R^1$ represents OH or acyloxy; $R^2$, $R^3$ and $R^4$, which may be the same or different, each represents H, OH or acyloxy; and $Z^1$ represents $OCOCH_2CH(CH_3)_2$ and $Z^2$ represents H, or $Z^1$ and $Z^2$ together represent =O) [hereinafter the compounds represented by formula (I) are referred to as Compounds (I)] in an aqueous solution containing no organic solvent, conjugating Compound (I) to a carrier substance by utilizing a substituent at the 3-position of the compound as a linker, and using the obtained conjugate as an immunogen.

The present inventors have also found that a monoclonal antibody with high specificity and affinity can be obtained by utilizing an acylated trichothecene mycotoxin as an immunogen.

Further, the present inventors have found that trichothecene mycotoxins in a sample can be determined in total, or in three major groups of DON, NIV and T-2, by utilizing the antibodies of the present invention after converting the trichothecene mycotoxins in the sample into compounds represented by formula (II):

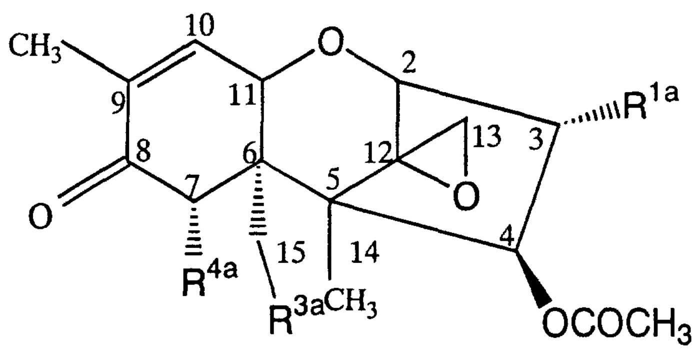

(II)

(wherein $R^{1a}$, $R^{3a}$ and $R^{4a}$, which may be the same or different, each represents OH or acyloxy) [hereinafter the compounds represented by formula (II) are referred to as Compounds (II)], compounds represented by formula (III):

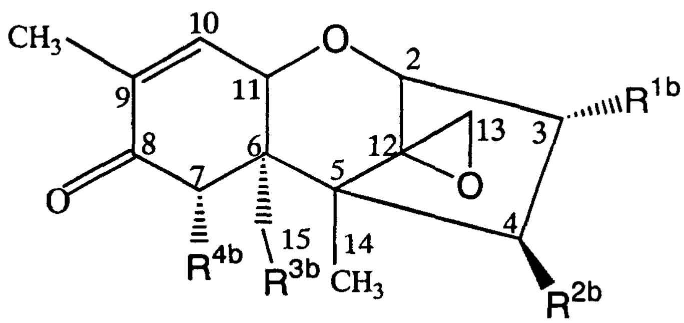

(III)

(wherein $R^{1b}$, $R^{3b}$ and $R^{4b}$, which may be the same or different, each represents OH or acyloxy; and $R^{2b}$ represents H, OH or acyloxy, provided that when $R^{2b}$ is H or OH, at least one of $R^{1b}$, $R^{3b}$ and $R^{4b}$ is acyloxy) [hereinafter the compounds represented by formula (III) are referred to as Compounds (III)] and compounds represented by formula (IV):

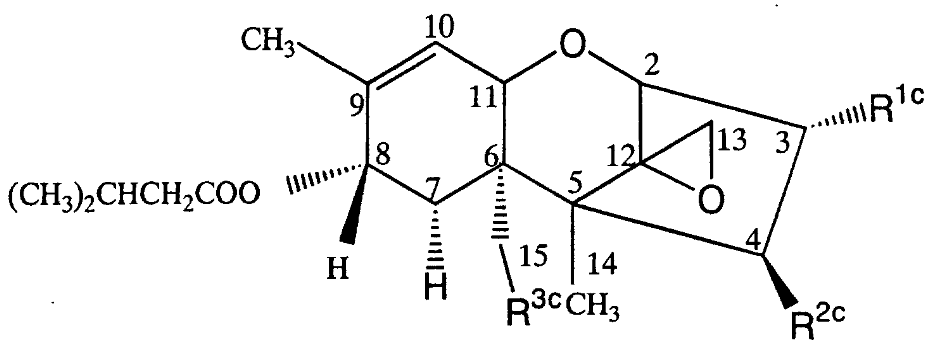

(IV)

(wherein $R^{1c}$, $R^{2c}$ and $R^{3c}$, which may be the same or different, each represents OH or acyloxy, provided that at least one of $R^{1c}$, $R^{2c}$ and $R^{3c}$ is acyloxy). The present invention has been completed on the basis of these findings.

In the definitions of the groups in the above formulae, the acyl in the acyloxy means a substituted or unsubstituted lower acyl group or a substituted or unsubstituted aromatic acyl group. The lower acyl group means a straight-chain or branched alkanoyl group having 1 to 12 carbon atoms, such as formyl, acetyl, propionyl, butyryl, isobutyryl, valeryl, isovaleryl, pivaloyl, hexanoyl, heptanoyl, octanoyl, decanoyl and dodecanoyl. The aromatic acyl group includes benzoyl and naphthoyl. Examples of the substituent in the substituted lower acyl group are hydroxy and carboxyl. Examples of the substituent in the substituted aromatic acyl group are lower alkyl, hydroxy, lower alkoxy, halogen and carboxyl. The lower alkyl moiety of the lower alkyl and the lower alkoxy means straight-chain or branched lower alkyl having 1 to 6 carbon atoms, such as methyl, ethyl, propyl, isopropyl, butyl, isobutyl, sec-butyl, tert-butyl, pentyl and hexyl. The halogen means fluorine, chlorine, bromine. or iodine.

Mycotoxins of NIV group include nivalenol (NIV), 4-acetylnivalenol, 3,4-diacetylnivalenol, 4,15-diacetylnivalenol, 3,4,15-triacetylnivalenol, 4,7,15-triacetylnivalenol and 3,4,7,15-tetraacetylnivalenol; mycotoxins of DON group include deoxynivalenol (DON), 3-acetyldeoxynivalenol, 3-acetyldeoxynivalenol, 15-acetyldeoxynivalenol, 3,15-diacetyldeoxynivalenol and 3,7,15-triacetyldeoxynivalenol; and mycotoxins of T-2 group include HT-2, T-2 and acetyl T-2.

The present invention relates to the following (1)-(40).

(1) A monoclonal antibody or a fragment thereof which has affinity for compounds represented by formula (II):

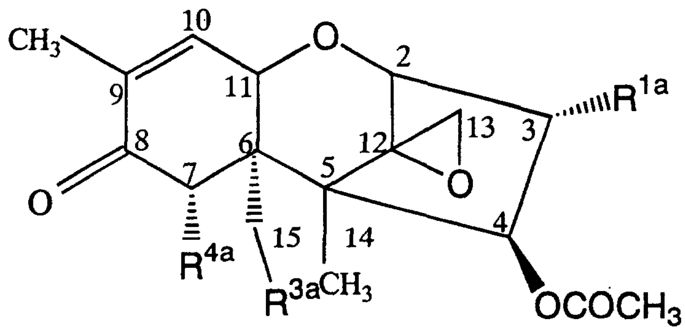

(II)

(wherein $R^{1a}$, $R^{3a}$ and $R^{4a}$, which may be the same or different, each represents OH or acyloxy) and which does not substantially react with compounds represented by formula (A):

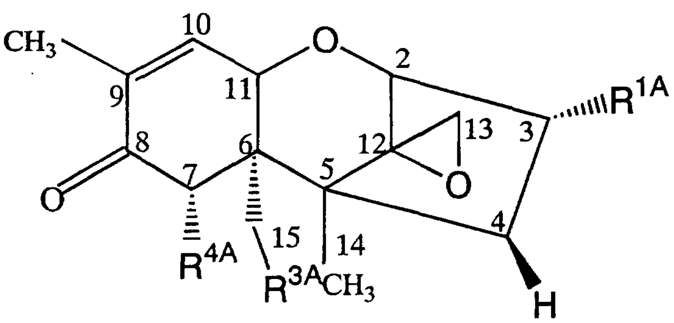

(A)

(wherein $R^{1A}$, $R^{3A}$ and $R^{4A}$, which may be the same or different, each represents OH or acyloxy) or compounds represented by formula (B):

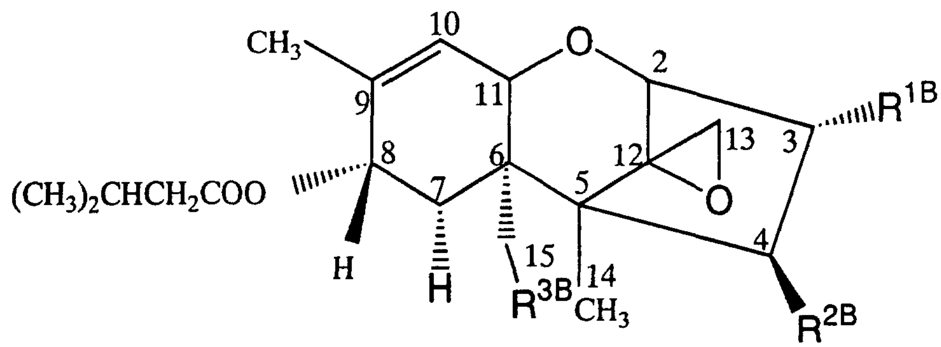

(B)

(wherein $R^{1B}$, $R^{2B}$ and $R^{3B}$, which may be the same or different, each represents OH or acyloxy), said affinity for the compounds represented by formula (II) decreasing in the order of Compound 1-1 > Compound 1-2 > Compound 1-3, wherein Compound 1-1 is a compound represented by formula (II) in which $R^{1a}$ and $R^{3a}$ are $OCOCH_3$ and $R^{4a}$ is OH, Compound 1-2 is a compound represented by formula (II) in which $R^{1a}$ and $R^{4a}$ are OH and $R^{3a}$ is $OCOCH_3$, and Compound 1-3 is a compound represented by formula (II) in which $R^{1a}$, $R^{3a}$ and $R^{4a}$ are $OCOCH_3$.

(2) The monoclonal antibody or a fragment thereof according to the above (1), wherein the monoclonal antibody is monoclonal antibody KTM-205 produced by hybridoma KTM-205.

(3) A monoclonal antibody or a fragment thereof which has affinity for compounds represented by formula (III):

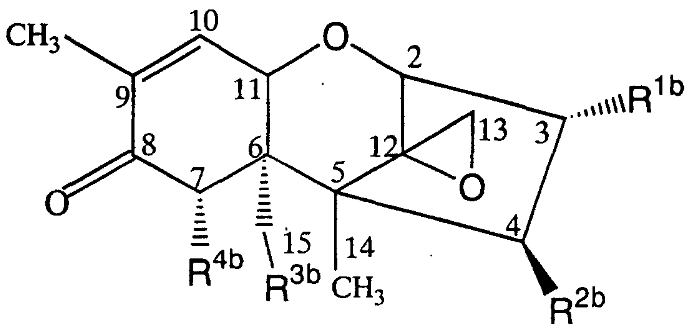

(III)

(wherein $R^{1b}$, $R^{3b}$ and $R^{4b}$, which may be the same or different, each represents OH or acyloxy; and $R^{2b}$ represents H, OH or acyloxy, provided that when $R^{2b}$ is H or OH, at least one of $R^{1b}$, $R^{3b}$ and $R^{4b}$ is acyloxy) and which does not substantially react with compounds represented by formula (C):

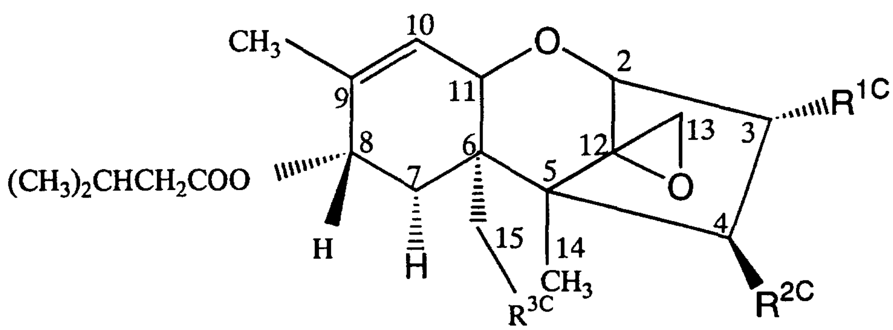

(C)

(wherein $R^{1C}$, $R^{2C}$ and $R^{3C}$, which may be the same or different, each represents OH or acyloxy), said affinity for the compounds represented by formula (III) decreasing in the order of Compound 2-1 > Compound 2-2 > Compound 2-3, wherein Compound 2-1 is a compound represented by formula (III) in which $R^{1b}$ and $R^{3b}$ are $OCOCH_3$, $R^{2b}$ is H and $R^{4b}$ is OH, Compound 2-2 is a compound represented by formula (III) in which $R^{1b}$, $R^{3b}$ and $R^{4b}$ are $OCOCH_3$ and $R^{2b}$ is H, and Compound 2-3 is a compound represented by formula (III) in which $R^{1b}$ and $R^{4b}$ are OH and $R^{2b}$ and $R^{3b}$ are $OCOCH_3$.

(4) The monoclonal antibody or a fragment thereof according to the above (3), wherein the monoclonal antibody is monoclonal antibody KTM-240 produced by hybridoma KTM-240.

(5) A monoclonal antibody or a fragment thereof which has affinity for compounds represented by formula (IV):

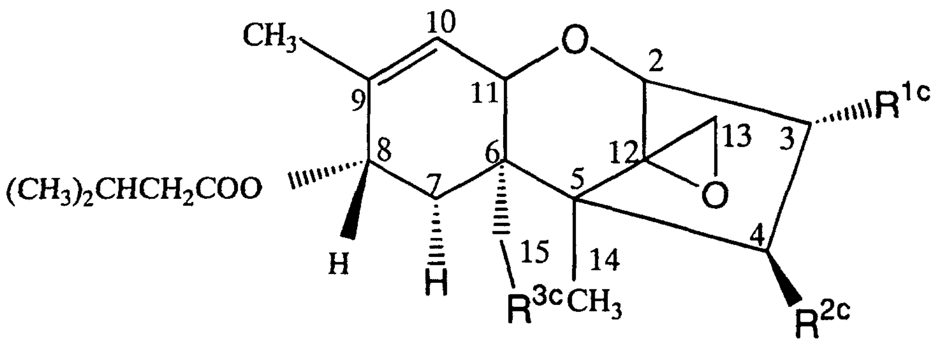

(IV)

(wherein $R^{1c}$, $R^{2c}$ and $R^{3c}$, which may be the same or different, each represents OH or acyloxy, provided that at least one of $R^{1c}$, $R^{2c}$ and $R^{3c}$ is acyloxy) and which does not substantially react with compounds represented by formula (D):

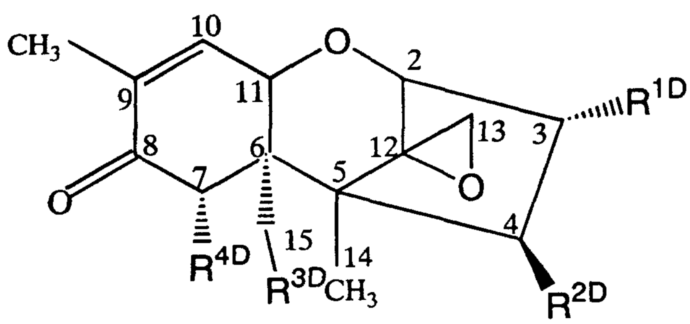

(D)

(wherein $R^{1D}$, $R^{3D}$ and $R^{4D}$, which may be the same or different, each represents OH or acyloxy, and $R^{2D}$ is H, OH or acyloxy), said affinity for the compounds represented by formula (IV) decreasing in the order of Compound 3-1 > Compound 3-2, wherein Compound 3-1 is a compound represented by formula (IV) in which $R^{1c}$ is OH and $R^{2c}$ and $R^{3c}$ are $OCOCH_3$, and Compound 3-2 is a compound represented by formula (IV) in which $R^{1c}$, $R^{2c}$ and $R^{3c}$ are $OCOCH_3$.

(6) The monoclonal antibody or a fragment thereof according to the above (5), wherein the monoclonal antibody is monoclonal antibody KTM-249 produced by hybridoma KTM-249.

(7) A hybridoma which is capable of producing the monoclonal antibody according to the above (1) or (2).

(8) A hybridoma which is capable of producing the monoclonal antibody according to the above (3) or (4).

(9) A hybridoma which is capable of producing the monoclonal antibody according to the above (5) or (6).

(10) The hybridoma according to the above (7) deposited with the National Institute of Bioscience and Human-Technology, Agency of Industrial Science and Technology under accession number FERM BP-6835.

(11) The hybridoma according to the above (8) deposited with the National Institute of Bioscience and Human-Technology, Agency of Industrial Science and Technology under accession number FERM BP-6836.

(12) The hybridoma according to the above (9) deposited with the National Institute of Bioscience and Human-Technology, Agency of Industrial Science and Technology under accession number FERM BP-6837.

(13) A process for producing a hybridoma which produces the monoclonal antibody according to any of the above (1) to (6), which comprises immunizing an animal by administering to the animal a substance prepared from a compound represented by formula (I):

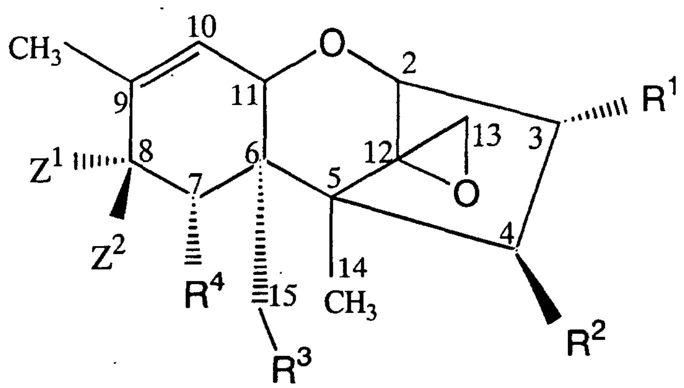

(I)

(wherein $R^1$ represents H or acyloxy; $R^2$, $R^3$ and $R^4$, which may be the same or different, each represents H, OH or acyloxy; and $Z^1$ represents $OCOCH_2CH(CH_3)_2$ and $Z^2$ represents H, or $Z^1$ and $Z^2$ together represent =O, provided that at least one of $R^1$, $R^2$, $R^3$ and $R^4$ is OH) by converting at least one of the hydroxyl groups therein to acyloxy and binding a carrier substance to the carbon at the 3-position thereof, and fusing an antibody-producing cell obtained from the immunized animal with a permanent growth cell to obtain the hybridoma.

(14) The process according to the above (13), wherein $R^2$ in formula (I) is acyloxy.

(15) The process according to the above (13), wherein the binding of the carrier substance to the carbon at the 3-position of a compound prepared from the compound represented by formula (I) by converting at least one of the hydroxyl groups therein to acyloxy is carried out by using a substituent at the 3-position as a linker.

(16) The process according to the above (13), wherein a compound prepared from the compound represented by formula (I) by converting at least one of the hydroxyl groups therein to a group represented by OR (wherein R represents a substituted or unsubstituted lower acyl group or a substituted or unsubstituted aromatic acyl group) is dissolved in a solvent which is not an organic solvent or which does not contain an organic solvent and then conjugated to the carrier substance.

(17) The process according to the above (16), wherein the solvent which is not an organic solvent or which does not contain an organic solvent is water.

(18) An immunoassay for determining a trichothecene mycotoxin in a sample, which comprises causing at least one of the monoclonal antibodies and fragments thereof according to the above (1) to (6) to act on the sample containing the trichothecene mycotoxin.

(19) An immunoassay for determining a trichothecene mycotoxin in a sample, which comprises converting at least one hydroxyl group in a compound represented by formula (I) having at least one hydroxyl group to a group represented by OR (wherein R represents a substituted or unsubstituted lower acyl group or a substituted or unsubstituted aromatic acyl group), and causing at least one of the monoclonal antibodies and fragments thereof according to the above (1) to (6) to act on the obtained compound.

(20) The immunoassay according to the above (18) or (19), wherein the trichothecene mycotoxin is selected from the group consisting of deoxynivalenol (DON), nivalenol (NIV), T-2 toxin (T-2) and derivatives thereof.

(21) A method for determining the total amount of DON, NIV, T-2 and derivatives thereof in a sample, which comprises calculating the total amount from the value obtained by the immunoassay according to the above (18) or (19) using the monoclonal antibody or a fragment thereof according to the above (3) or (4) and the value obtained by the immunoassay according to the above (18) or (19) using the monoclonal antibody or a fragment thereof

according to the above (5) or (6).

(22) A method for determining NIV and its derivatives in a sample, which comprises carrying out the immunoassay according to the above (18) or (19) using the monoclonal antibody or a fragment thereof according to the above (1) or (2).

(23) A method for determining DON, NIV and derivatives thereof in a sample, which comprises carrying out the immunoassay according to the above (18) or (19) using the monoclonal antibody or a fragment thereof according to the above (3) or (4).

(24) A method for determining DON and its derivatives in a sample, which comprises calculating the amount of DON and its derivatives from the value obtained by the immunoassay according to the above (18) or (19) using the monoclonal antibody or a fragment thereof according to the above (3) or (4) and the value obtained by the immunoassay according to the above (18) or (19) using the monoclonal antibody or a fragment thereof according to the above (1) or (2).

(25) A method for determining T-2 and its derivatives in a sample, which comprises carrying out the immunoassay according to the above (18) or (19) using the monoclonal antibody or a fragment thereof according to the above (5) or (6).

(26) The immunoassay according to the above (18) or (19), wherein the immunoassay is selected from the group consisting of radioimmunoassay, enzyme immunoassay, fluoroimmunoassay and luminescence immunoassay.

(27) The immunoassay according to the above (18) or (19), wherein the immunoassay is selected from the group consisting of competitive immunoassay and sandwich immunoassay.

(28) A reagent for immunoassay for determining a trichothecene mycotoxin, comprising at least one of the monoclonal antibodies and fragments thereof according to the above (1) to (6) as an active ingredient.

(29) A kit for immunoassay for determining a trichothecene mycotoxin, comprising the reagent according to the above (28) and an antigen-immobilized solid phase plate.

(30) A kit for immunoassay for determining a trichothecene mycotoxin, comprising the reagent according to the above (28), an antigen-immobilized solid phase plate, a labeled antibody or antibody fragment which reacts with the monoclonal antibody or a fragment thereof according to any of the above (1) to (6), and a reagent for detecting the label of said antibody or antibody fragment.

(31) A kit for immunoassay for determining a trichothecene mycotoxin, comprising an antigen-immobilized solid phase plate, the monoclonal antibody or a fragment thereof according to any of the above (1) to (6) which is labeled, and a reagent for detecting the label of said antibody or antibody fragment.

(32) A kit for immunoassay for determining a trichothecene mycotoxin, comprising the reagent according to the above (28) and a solution for the pretreatment of a sample to convert a hydroxyl group in a compound represented by formula (I) to a group represented by OR (wherein R has the same significance as defined above).

(33) A kit for immunoassay for determining a trichothecene mycotoxin, comprising the reagent according to the above (28), an antigen-immobilized solid phase plate, and a solution for the pretreatment of a sample to convert a hydroxyl group in a compound represented by formula (I) to a group represented by OR (wherein R has the same significance as defined above).

(34) A kit for immunoassay for determining a trichothecene mycotoxin, comprising the reagent according to the above (28), an antigen-immobilized solid phase plate, a labeled antibody or antibody fragment which reacts with the monoclonal antibody or a fragment thereof according to any of the above (1) to (6), a reagent for detecting the label of said antibody or antibody fragment, and a solution for the pretreatment of a sample to convert a hydroxyl group in a compound represented by formula (I) to a group represented by OR (wherein R has the same significance as defined above).

(35) A kit for immunoassay for determining a trichothecene mycotoxin, comprising an antigen-immobilized solid

phase plate, the monoclonal antibody or a fragment thereof according to any of the above (1) to (6) which is labeled, a reagent for detecting the label of said antibody or antibody fragment, and a solution for the pretreatment of a sample to convert a hydroxyl group in a compound represented by formula (I) to a group represented by OR (wherein R has the same significance as defined above).

(36) A method for determining a trichothecene mycotoxin in a sample, which comprises treating the sample containing the trichothecene mycotoxin with a solution containing an organic solvent to extract the trichothecene mycotoxin from the sample, and determining the extracted trichothecene mycotoxin by the immunoassay according to the above (18) or (19).

(37) The method according to the above (36), wherein the organic solvent is a water-soluble organic solvent.

(38) The method according to the above (36) or (37), wherein the water-soluble organic solvent is at least' one member selected from the group consisting of methyl alcohol, ethyl alcohol, propyl alcohol, acetonitrile, dimethyl sulfoxide and dimethylformamide.

(39) A method for detecting a microorganism producing a trichothecene mycotoxin in a sample by immunoassay, which comprises inoculating the sample containing the microorganism producing the trichothecene mycotoxin into a medium, culturing the microorganism in the medium, and causing at least one of the monoclonal antibodies and fragments thereof according to the above (1) to (6) to act on the trichothecene mycotoxin produced in the culture.

(40) A method for identifying a microorganism producing a trichothecene mycotoxin in a sample by immunoassay, which comprises inoculating the sample containing the microorganism producing the trichothecene mycotoxin into a medium, culturing the microorganism in the medium, and causing at least one of the monoclonal antibodies and fragments thereof according to the above (1) to (6) to act on the trichothecene mycotoxin produced in the culture.

1. Process for Producing Monoclonal Antibodies for Trichothecene Mycotoxins

The process for producing the monoclonal antibodies for trichothecene mycotoxins of the present invention is described below.

Monoclonal antibodies can be prepared by a process which comprises fusing an antibody-producing cell obtained from an animal immunized by administration of an immunogen with a permanent growth cell such as a myeloma cell to produce a hybridoma, culturing the hybridoma or administering the hybridoma to an animal to cause ascites tumor, and separating and purifying a monoclonal antibody from the resulting culture or ascites.

In order to obtain the monoclonal antibodies for trichothecene mycotoxins of the present invention, it is necessary to use, as an immunogen, a conjugate of an antigen for immunization and a high molecular weight carrier (referred to also as carrier substance). The antigens for immunization may be obtained by purification from samples or by chemical synthesis. In this procedure, acylated trichothecene mycotoxins are used. The desired monoclonal antibody according to the invention can be efficiently obtained by using, as an immunogen, a conjugate prepared by binding a high molecular weight carrier to an acylated trichothecene mycotoxin utilizing a substituent at the 3-position of the mycotoxin as a linker.

As the antigen for immunization, compounds prepared by converting a hydroxyl group in the compounds represented by formula (I) to a group represented by OR (wherein R has the same significance as defined above) are used. Preferably, the compound represented by formula (b-1):

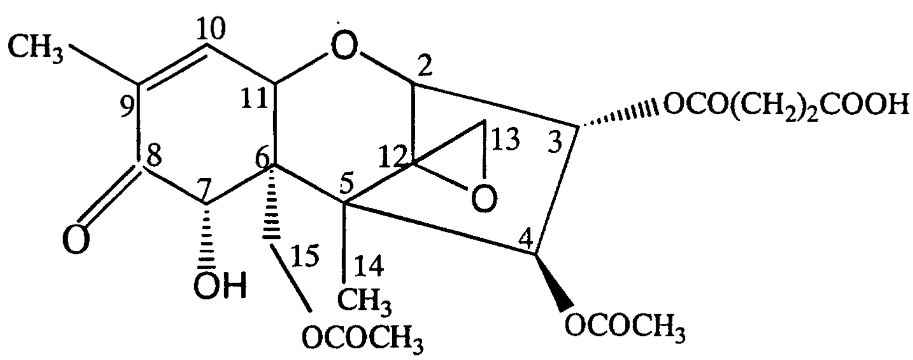

and the compound represented by formula (b-2):

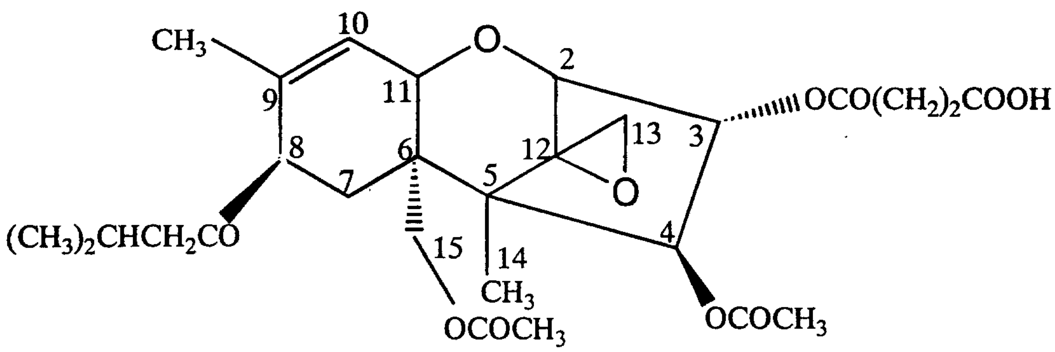

are used.

The samples can be prepared by inoculating strains of Fusarium graminearum, F. culmorum, F. sporotrichioides, etc. into suitable media and culturing them at around room temperature for about 20 days, followed by purification by appropriate means. Suitable media include commercially available media such as potato-dextrose medium, and media prepared, for example, by wetting polished rice with distilled water and then sterilizing it in an autoclave. Purification can be carried out, for example, by extracting the obtained culture with acetonitrile/water (3:1 v/v) and passing the extract through a column packed with layers of Florisil and anhydrous sodium sulfate or layers of silica gel and anhydrous sodium sulfate, or by means of recrystallization. The samples may also be obtained by purification from cereals contaminated with molds. Purification can be carried out by any methods capable of purifying the desired substance, for example, by using TLC plate or by HPLC. Also useful are commercially available samples.

As the high molecular weight carrier, any high molecular weight substance can be used so far as it has a reactive group for condensation with a carboxyl group, an amino group, a hydroxyl group, or the like in the antigen for immunization and is capable of endowing the antigen for immunization with immunogenicity or enhancing the immunogenicity of the antigen when bound thereto.

Suitable high molecular weight substances include proteins such as bovine serum albumin (BSA), globulin, keyhole limpet hemocyanin (KLH) and thyroglobulin, polysaccharides such as dextran and Sepharose, latex particles comprising polystyrene and acrylic rubber, polynucleic acids such as polyuridylic acid and polyadenylic acid, and synthetic high molecular weight substances such as MAP (multiple antigen peptide). The high molecular weight substance can be conjugated to a trichothecene mycotoxin by various methods such as methods using an amino group described in Nobuo Sakato, Meneki Jikken Sosaho (Operations in Immunological Experiments), P. 151, Shunsuke Migita, et al. edit., Nankodo (1995) (the carbodiimide method, the glutaraldehyde method and the diisocyanate method), methods using a carboxyl group (the active ester method, the acid anhydride mixture method and the acylazide method), methods using an SH group (MBS method and SPDP method) and methods using a hydroxyl group (the cyanogen bromide method and the periodic acid oxidation method). In these procedures, it is important to dissolve a trichothecene mycotoxin in an aqueous medium, not in an organic solvent or a solution containing an organic solvent, in order to allow it to exert sufficient antigenecity in an animal.

The immunogen is administered to an animal such as mouse, rat, hamster, rabbit, guinea pig, goat, sheep, horse or fowl, preferably mouse, rat or hamster.

Immunization can be carried out according to the method described in Saido and Toyoshima, Shin Seikagaku Jikken Koza (New Lectures on Experiments in Biochemistry), 1:389 (1990) Tokyo Kagaku Dojin, etc. For example, the immunogen is emulsified with complete or incomplete Freund's adjuvant, and the resulting emulsion is administered to an animal intraperitoneally, subcutaneously or intramuscularly. Administration of 1.0 to 300 μg of immunogen is made twice or more, preferably 2 to 4 times at regular intervals of 7 to 30 days, preferably 12 to 16 days to complete immunization.

Antibody-producing cells can be obtained from the spleen, lymph nodes, peripheral blood, etc. of an immunized animal. Antibody-producing cells can also be obtained by the so-called "in vitro immunization", that is, by directly immunizing cells responsible for antibody production which have been collected from the spleen, lymph nodes, peripheral blood, etc. of an unimmunized animal [Arai and Ohta, Experimental Medicine, 6:43 (1988)].

There is no specific restriction as to the myeloma cells to be used for fusion with antibody-producing cells, but it is preferred to use cell lines derived from the same species of animal as the one from which the antibody-producing cells are derived.

It is also preferred to use myeloma cells having specific drug markers in order to efficiently select cells which have been appropriately fused. For example, 8-azaguanine-resistant myeloma cells are preferably used because they can not grow in a medium containing hypoxanthine, aminopterin and thymidine (HAT medium), but fused cells resulting from fusion of these myeloma cells with normal cells can grow in HAT medium and thus can be distinguished from unfused myeloma cells. Specific examples are P3 x 63-Ag, P3 x -Ag8-U1 and SP2/O-Ag14. These myeloma cells are available from the Cell Bank of the Institute of Physical and Chemical Research.

Cell fusion can be carried out by applying the method developed by Kohler and Milstein [Nature, 256:495 (1975)] and its rapidly and variously developed modifications. In a method generally employed, antibody-producing cells and myeloma cells are mixed in a ratio of 10 to 3:1 and treated with 30 to 50% polyethylene glycol (average molecular weight: 1500 to 6000) as a fusing agent. Fusion can also be carried out by electroporation [Ohkouchi, et al., Experimental Medicine, 6:50 (1988)].

The cells which have been subjected to the cell fusion treatment are suspended in a selective medium and cultured in a culture vessel favorable for selecting the desired cells such as a 96-well culture plate to selectively grow fused cells.

The term "selective medium" refers to a medium which allows the selective growth of cells having a specific drug marker or the like. For example, the fused cells resulting from the fusion of antibody-producing cells with the P3 x 63-Ag myeloma cells can be efficiently selected based on the 8-azaguanine resistance of the myeloma cells by using HAT selective medium (RPMI-1640 medium supplemented with 10% FCS and containing $1 \times 10^{-4}$ mol/l hypoxanthine, $4 \times 10^{-7}$ mol/l aminopterin and $2 \times 10^{-5}$ mol/l thymidine) or the like.

The cells producing antibodies for the target substance are selected from the selectively grown fused cells by detecting the presence of desired antibodies in the culture supernatant of fused cells by a method such as enzyme immunoassay or radioimmunoassay. From the selected cells are obtained single cell colonies by the limiting dilution method, the soft agar culture method or the like to obtain monoclonal antibody-producing hybridomas.

The hybridomas are cultured in an appropriate medium, or are intraperitoneally transplanted into an animal and allowed to grow in its ascites. The monoclonal antibodies can be obtained from the collected culture or ascites. The antibodies in the culture or ascites can be used after purification, if necessary. Purification can be carried out by various methods, e.g., salting-out fractionation using ammonium sulfate, ion exchange chromatography, gel filtration column chromatography, affinity column chromatography using protein A or protein G, and affinity column chromatography using antigen-immobilized gel, alone or in combination.

Anti-trichothecene mycotoxin monoclonal antibodies can be obtained by the above-described process. The thus obtained monoclonal antibodies include monoclonal antibodies designated KTM-205, KTM-240 and KTM-249, respectively.

Monoclonal antibody KTM-205 has affinity for the compounds represented by formula (II) and does not substantially react with the compounds represented by formula (A) or (B). Its affinity for Compound 1-1, Compound 1-2 and Compound 1-3 is in the decreasing order of Compound 1-1 > Compound 1-2 > Compound 1-3.

Monoclonal antibody KTM-240 has affinity for the compounds represented by formula (III) and does not substantially react with the compounds represented by formula (C). Its affinity for Compound 2-1, Compound 2-2 and Compound 2-3 is in the decreasing order of Compound 2-1 > Compound 2-2 > Compound 2-3.

Monoclonal antibody KTM-249 has affinity for the compounds represented by formula (IV) and does not substantially react with the compounds represented by formula (D). Its affinity for Compound 3-1 and Compound 3-2 is in the decreasing order of Compound 3-1 > Compound 3-2.

2. Method for Determination of Trichothecene Mycotoxins

Trichothecene mycotoxins contained in a sample may be determined by any of the immunoassays capable of determination of trichothecene mycotoxins, but can be advantageously determined by immunoassay using the monoclonal antibodies or monoclonal antibody fragments of the present invention.

The samples containing mycotoxins include all of the substances that may possibly contain mycotoxins, for example, crops, products obtained by processing crops, and environmental factors for cultivation of crops.

The crops may be any of the farm crops, for example, rice, barley, wheat, rye, oats, beans, maize and potatoes. The crops include those growing in cultivation fields such as farms and those on the market.

The products obtained by processing crops include all foods and beverages obtained by processing crops.

The samples containing mycotoxins also include feeds, that is, foods for livestock, meat, milk and eggs of livestock such as pigs, cattle and fowl taking feeds, and products obtained by processing such meat, milk and eggs.

The environmental factors for cultivation of crops include various factors constituting the environment for crops, for example, inorganic factors such as moisture and soil, microorganisms living in adhesion to the inorganic factors, spores of microorganisms floating in the air, and organic residues such as stumps or stubbles of plants.

Most of the above-mentioned samples containing mycotoxins are solids. Therefore, the samples may be

subjected to pretreatment to recover mycotoxins from the samples and to make liquid samples suitable for general immunoassays, unless it is not necessary to extract mycotoxins. Pretreatment is carried out in the following manner.

Recovery of mycotoxins can be carried out by treating the samples containing mycotoxins, as such or after being disrupted, with an organic solvent to extract mycotoxins and recovering the extract.

The samples may be disrupted by any methods, so far as mycotoxins are not decomposed or do not disappear by the disruption treatment. Suitable methods include grinding with a mortar and pestle, trituration by sonication or by using a mill or a hammer, and mincing with a cutter.

The samples, as such or after being disrupted by the above methods, are treated with an organic solvent, for example, by directly immersing the samples in an organic solvent. Extraction efficiency may be enhanced by operation such as stirring and sonication.

Though any organic solvents can be used, preferred organic solvents are water-soluble ones, such as methyl alcohol, ethyl alcohol, propyl alcohol, acetonitrile, dimethyl sulfoxide and dimethylformamide. The content of organic solvent in the whole extract is 25% or more, preferably 50% or more, more preferably 75% or more.

Trichothecene mycotoxins extracted with an organic solvent can be recovered by evaporating the solvent or by passing the extract through a column.

The pretreatment process according to the present invention may further comprise the step of converting part of the trichothecene mycotoxins into their derivatives, for example, the step of partial acylation, preferably partial acetylation. The step of acetylation is described below.

Acetylation is carried out by dissolving the subject compound in an appropriate solvent and adding acetic anhydride and a base to cause reaction. Suitable solvents include halogen-containing organic solvents such as dichloromethane and chloroform, and general solvents which do not react with acetic anhydride such as diethyl ether, DMF, DMSO and dichloromethane.

Bases useful in the reaction include organic bases such as pyridine and triethylamine, and inorganic bases such as sodium bicarbonate and potassium carbonate. Preferred is pyridine, which serves both as a base and a solvent.

In order to acetylate only the desired part in the partial acetylation, it is necessary to control the concentration of acetic anhydride, the reaction temperature and the reaction time. It is preferred that the sample be well dried prior to the acetylation, that the reaction temperature be not high and kept constant during the reaction, and that the reaction time be constant.

Further, it is necessary to force the reaction to terminate at an appropriate time in order to avoid allowing the reaction to proceed excessively.

The concentration of acetic anhydride is 0.1 to 10000 times, preferably 0.5 to 10 times the weight of the dried sample, and needs to be adjusted according to the amount of organic solvent added.

The reaction may be carried out at any temperature between ice-cooling and the boiling point of the solvent employed, but is carried out preferably at 30 to 50°C, more preferably 45°C. These acetylation conditions need not be specifically restricted so far as the object can be attained. Typically, when 20 mg of the dried sample is used, the reaction is carried out at 45°C for about 45 minutes using 50 µl of pyridine and 25 µl of acetic anhydride.

The reaction may be terminated immediately after the start of reaction or may be carried out for a period of one week, but from a practical point of view, it is preferred to terminate the reaction within one hour. The reaction can be terminated by removal of base and acetic anhydride or by addition of alkali. The base and acetic anhydride can be readily removed by evaporation. As the alkali, an aqueous solution of sodium bicarbonate is preferably used.

The above acetylation conditions need not be specifically restricted so far as the object can be attained.

Among the above-mentioned samples containing trichothecene mycotoxins, those which may possibly contain microorganisms producing trichothecene mycotoxins, for example, environmental factors for cultivation of crops can be assayed without conducting one or both of the step of extraction with an organic solvent and the step of partial acetylation. That is, after such samples are directly inoculated into a culture medium to grow microorganisms, the compounds of formulae (II), (III) and (IV) produced in the medium can be determined by an immunoassay. It is also possible to inoculate such samples on a selective medium such as Komada medium (1 g of $K_2HPO_4$, 0.5 g of KCl, 0.5 g of $MgSO_4 \cdot 7H_2O$, 0.01 g of Fe-EDTA, 2 g of L-asparagine, 20 g of D-galactose, 1 g of pentachloronitrobenzene 75% hydrate, 0.5 g of sodium cholate, 1 g of $NaB_4O_7 \cdot 10H_2O$, 0.3 g of streptomycin sulfate, 15 g of agar, and 1000 ml of distilled water, pH 3.8 to 4.0), inoculate the microorganisms which grow on the selective medium into a culture medium, and then determine the compounds of formulae (II), (III) and (IV) produced in the medium by an immunoassay.

Further, spores of microorganisms floating in the air can be captured by putting a Petri dish containing Komada medium at about 90 cm above the ground and leaving it open to the air for a certain period. The dish in which the spores have been captured is subjected to culturing at room temperature (20 to 25°C) for about one week, and colonies appearing thereby can be used as samples.

Any culture media can be used so long as the microorganisms can grow therein. Preferred is a medium prepared by leaving polished rice with a certain amount of distilled water in a vessel capable of autoclaving for a certain period for wetting, followed by sterilization in an autoclave. The amount of distilled water is preferably 1 to 10 times

that of polished rice. The rice and water are left to stand preferably for 15 minutes to a half day.

The immunoassay of the present invention can be carried out by any of the known immunoassay methods.

Suitable methods include various sensitive immunoassays such as radioimmunoassay using radioisotopes for labeling, enzyme immunoassay using enzymes, fluoroimmunoassay using fluorescent substances and luminescence immunoassay using luminescent substances.

In an immunoassay, the amount of antibody or antigen is determined using an antigen or antibody labeled as described above. In the present invention, any methods for detection or determination of antigens can be employed for immunoassay, but competitive immunoassay and sandwich immunoassay are most suitable.

Further, various modifications of the above methods are known. For example, modifications of competitive immunoassay include (1) a method using competition between a labeled antigen and an antigen in a sample or standard substance for binding to an antibody or antibody fragment, (2) a method using competition between an antigen in a liquid phase sample or standard substance and an immobilized antigen for binding to a labeled antibody or antibody fragment, and (3) a method using competition between a labeled antigen and an antigen in a sample or standard substance for binding to an immobilized antibody or antibody fragment.

Sandwich immunoassays generally comprise reacting a primary antibody immobilized on a solid phase support (i.e., an antibody or antibody fragment immobilized on an appropriate solid phase support such as beads, tube or plate) with an antigen in a standard substance or sample, reacting the antigen bound to the immobilized antibody (or antibody fragment) with a secondary antibody (or antibody fragment), and detecting the formed tripartite complex of immobilized antibody (or antibody fragment)-antigen-secondary antibody (or antibody fragment) by some appropriate means. Generally, detection is carried out by labeling the secondary antibody (or antibody fragment) with various labeling substances for detection, for example, radioisotopes, enzymes, fluorescent substances, luminescent substances and metals. These assays are mainly used for determining antigens in solutions, but are also useful in qualitative or quantitative assays for trichothecene mycotoxins which are present in tissues and cells and on membrane filters for tests such as nitrocellulose membrane and nylon membrane.

The present invention provides a reagent or kit for immunoassay for determining trichothecene mycotoxins. The kit consists of combinations of instruments and/or reagents and may vary in constitution or form so far as it comprises substances which are essentially identical with the constituents described below or part thereof.

The reagent for immunoassay according to the present invention comprises the monoclonal antibody of the present invention or a fragment thereof as an active ingredient. The kit according to the present invention for use in assays of samples which require the acylation step comprises a solution for the pretreatment of samples to convert a hydroxyl group in a compound represented by formula (I) to a group represented by OR (wherein R has the same significance as defined above) and the monoclonal antibody of the present invention or a fragment thereof.

The above kit for immunoassay according to the present invention may further comprise, as may be required, a diluent for a sample, a plate for determination, a labeled secondary anti-mouse antibody, a reagent for detection of a labeling substance, a standard substance, etc.

Suitable diluents for a sample include solutions comprising a surfactant, a buffer and a protein such as BSA or casein. As the plate for determination, an antigen-immobilized 96-well microtiter plate of polystyrene or the like can be used. Suitable labeled secondary anti-mouse antibodies include antibodies having affinity for the anti-trichothecene mycotoxin monoclonal antibody or antibody fragment of the present invention, e.g., a rabbit anti-mouse immunoglobulin antibody, which are labeled with labeling enzymes such as horseradish peroxidase (HRP), bovine small intestine alkaline phosphatase and β-galactosidase, and mixed with a buffer, a protein such as BSA or casein, a preservative, etc. The reagents for detection of labeling substances may comprise various components according to the labeling enzymes mentioned above; for example, when horseradish peroxidase is used, the reagent mainly comprises tetramethylbenzidine, orthophenylenediamine, etc. In the reagent according to the present invention, the anti-trichothecene mycotoxin monoclonal antibody or antibody fragment of the present invention directly labeled with a labeling enzyme may also be employed. Further, the kit for immunoassay according to the present invention needs not to contain all of the components mentioned above and may comprise additional components.

3. Method for Identification of Microorganisms Producing Trichothecene Mycotoxins

The samples containing microorganisms producing trichothecene mycotoxins include environmental factors for cultivation of crops, for example, moisture, soil, air, spores floating in the air, and organic residues such as stumps or stubbles of plants.

The microorganisms producing trichothecene mycotoxins form perithecia at residual plants in cultivation fields, for example, organic residues such as rice stubbles. When environmental conditions such as precipitation and high humidity are fulfilled, ascospores of the microorganisms scatter in the air to infect crops during the stage of blooming or ripening.

Therefore, it is critically important to know the condition of contamination of cultivation fields by the microorganisms producing trichothecene mycotoxins and their ability to produce mycotoxins in order to foresee the occurrence of mycotoxin damage and to take countermeasures.

The trichothecene mycotoxin-producing strains, when cultured in a medium, specifically produce trichothecene mycotoxins including the compounds represented by formulae (II), (III) and (IV) in the medium. The kind and amount of trichothecene mycotoxins produced vary with the trichothecene mycotoxin-producing strain.

Accordingly, the presence of a trichothecene mycotoxin-producing microorganism in a sample can be confirmed by detecting the presence of the above compounds in the culture medium of the sample according to the immunoassay described in the above 2. Further, a strain can be identified by qualitatively and quantitatively analyzing the trichothecene mycotoxins produced by the strain according to the immunoassay described in the above 2.

It is further possible to know the condition of environmental pollution in cultivation fields by identifying the trichothecene mycotoxin-producing microorganisms existing in the fields according to the immunoassay described in the above 2.

The above samples are directly inoculated into a culture medium to grow microorganisms, and the compounds represented by formulae (II), (III) and (IV) in the medium are determined to detect the contamination by the microorganisms producing trichothecene mycotoxins. It is also possible to inoculate the samples on a selective medium such as Komada medium (1 g of $K_2HPO_4$, 0.5 g of KCl, 0.5 g of $MgSO_4 \cdot 7H_2O$, 0.01 g of Fe-EDTA, 2 g of L-asparagine, 20 g of D-galactose, 1 g of pentachloronitrobenzene 75% hydrate, 0.5 g of sodium cholate, 1 g of $NaB_4O_7 \cdot 10H_2O$, 0.3 g of streptomycin sulfate, 15 g of agar, and 1000 ml of distilled water, pH 3.8 to 4.0), inoculate the microorganisms which grow by culturing on the selective medium into a culture medium, and then determine the compounds of formulae (II), (III) and (IV) produced in the medium to detect the microorganism contamination. Further, spores of microorganisms floating in the air can be captured by putting a Petri dish containing Komada medium at about 90 cm above the ground and leaving it open to the air for a certain period. The dish in which the spores have been captured is subjected to culturing at room temperature (20 to 25°C) for about one week, and colonies appearing thereby can be advantageously utilized.

As the culture medium, various media can be used. Preferred is a medium prepared by leaving polished rice with a certain amount of distilled water in a vessel capable of autoclaving for a certain period for wetting, followed by sterilization in an autoclave.

## Brief Description of the Drawings

Fig. 1 shows a calibration curve for the determination of trichothecene mycotoxins using KTM-205.
Fig. 2 shows a calibration curve for the determination of trichothecene mycotoxins using KTM-240.
Fig. 3 shows a calibration curve for the determination of trichothecene mycotoxins using KTM-249.
Fig. 4 shows a calibration curve for the determination of trichothecene mycotoxins using KTM-240 and KTM-249.
Fig. 5 shows the correlation between the amounts of trichothecene mycotoxins in samples as determined by ELISA according to the present invention and those in the same samples as determined by GC-MS.
Fig. 6 shows the result of assay for trichothecene mycotoxins in a culture of F. graminearum obtained by culturing it in a rice medium using KTM-240.
Fig. 7 shows the result of assay for trichothecene mycotoxins in cultures of strains isolated from rice stalks from different cultivation fields.

## Best Modes for Carrying Out the Invention

The present invention is described below by referring to examples.

### Example 1 (Production of Monoclonal Antibodies)

a) Compounds

N7769 (Sigma Chemical Co.) was used as the compound of formula (a-1):

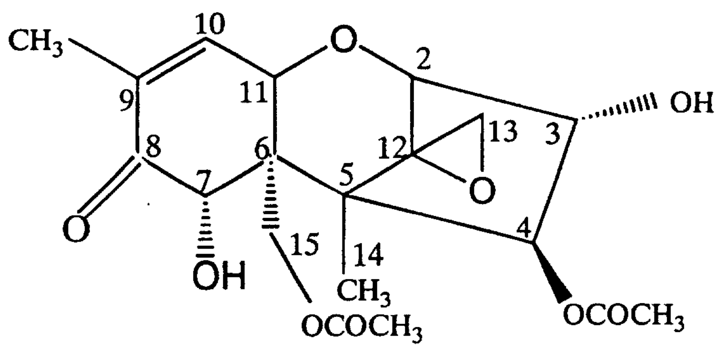

and T4887 (Sigma Chemical Co.) was used as the compound of formula (a-2):

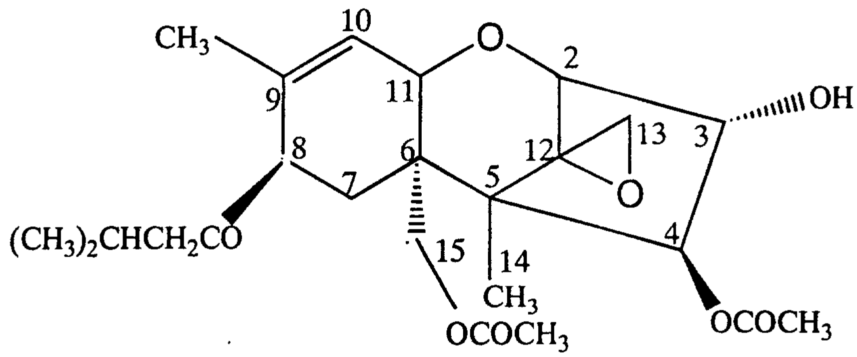

b) Preparation of Antigens for Immunization

The compound of formula (a-1) (4,15-diacetylnivalenol) and the compound of formula (a-2) (T-2) (50 mg each) were respectively put into 7-ml test tubes with screw caps. After 1 ml of dry pyridine and 35 mg of an acid anhydride were added to each test tube, the mixtures were subjected to reaction at 100°C for 3 hours in Metal Block Bath (YH-121, Yamato Kagaku). Each reaction mixture was concentrated to dryness to remove pyridine, followed by addition of 4 ml of chloroform and washing with four 1 ml portions of water. The chloroform layer was dehydrated over anhydrous sodium sulfate. The resulting solutions were subjected to silica gel thin layer chromatography, gas chromatography and gas chromatography-mass spectrometry (GC-MS) to confirm the presence of the compound of formula (b-1) (4,15-diacetylnivalenol 3-hemisuccinate) and the compound of formula (b-2) (T-2 3-hemisuccinate) in the respective solutions, and then concentrated to dryness under reduced pressure.

The obtained dry concentrates containing the compounds of formulae (b-1) and (b-2) (0.5 mg each) were respectively dissolved in 50 μl of 10 mM phosphate buffer (pH 7.4) containing 140 mM NaCl (hereinafter abbreviated as PBS) with the aid of addition of 0.01% Triton X-100 and a ultrasonic generator of probe type (Model UR-200P, Tommy Seiko). Each of the solutions was mixed with 0.5 ml of an aqueous solution of keyhole limpet hemocyanin (hereinafter abbreviated as KLH) (20 mg/ml). After each mixture was adjusted to pH 7.5, 20 mg of EDC [1-ethyl-3-(3-dimethylaminopropyl)carbodiimide] was added thereto, followed by reaction at room temperature for 6 hours. After the completion of reaction, dialysis was carried out against PBS to obtain aqueous solutions of trichothecene mycotoxin derivative [formula (b-1)]-KLH and trichothecene mycotoxin derivative [formula (b-2)]-KLH as dialyzates. The protein concentrations in the aqueous solutions were determined by means of Lowry Protein Assay Kit (Bio-Rad) using bovine serum albumin (hereinafter abbreviated as BSA) as a standard. The above procedure was repeated using BSA instead of KLH, whereby solutions of trichothecene mycotoxin derivative [formula (b-1)]-BSA conjugate and trichothecene mycotoxin derivative [formula (b-2)]-BSA conjugate were obtained.

c) Preparation of Monoclonal Antibodies

Six-week-old Balb/c male mice were injected subcutaneously at the back with a 1:1 mixture of complete Freund's adjuvant and trichothecene mycotoxin derivative [formula (b-1) or formula (b-2)]-KLH in an amount of 0.1 mg per animal. Further administration of the above 1:1 mixture (0.1 mg/animal) was made subcutaneously at the back of mice twice at 3-week intervals. After 3 weeks, trichothecene mycotoxin derivative [formula (b-1) or formula (b-2)]-KLH dissolved in PBS (0.1 mg/animal) was administered to the mice through the tail vein, and 3 days thereafter, antibody-producing cells were obtained from the spleen in the following manner.

The spleen was aseptically excised from an immunized animal, loosened in serum-free RPMI-1640 medium (Nissui Pharmaceutical Co., Ltd.) and passed through a 100-mesh net to liberate spleen cells. The spleen cells were suspended in a hypotonic solution to dissolve erythrocytes therein, and washing with centrifugation was carried out three times using serum-free RPMI-1640 medium to obtain antibody-producing cells.

On the other hand, P3X-Ag8-U1 mouse myeloma cells were cultured in RPMI-1640 medium containing 10% fetal calf serum (FCS). The cells were collected at the logarithmic growth phase and washed with centrifugation three times using serum-free RPMI-1640 medium.

The obtained antibody-producing cell suspension and P3X-Ag8-U1 mouse myeloma cell suspension were mixed at a ratio of 10:1 and centrifuged at 1200 rpm for 5 minutes to remove the culture liquor. To the obtained cells was slowly added 1 ml of 50% polyethylene glycol 1500 solution (Boehringer Mannheim GmbH) and then was gradually added 50 ml of serum-free RPMI-1640 medium, followed by centrifugation at 1200 rpm for 5 minutes to remove the medium. The obtained cells were suspended in HAT medium (RPMI-1640 medium supplemented with 10% FCS and containing $1 \times 10^{-4}$ M hypoxanthine, $4 \times 10^{-7}$ M aminopterin and $2 \times 10^{-5}$ M thymidine) at a density of $1 \times 10^{6}$ cells/ml, and the cell suspension was put into wells of a 96-well microtiter plate in an amount of 200 μl per well. The cells were incubated at 37°C in a 5% $CO_2$ incubator, and after 10 days of incubation, hybridoma colonies were observed in all of the wells.

The culture supernatants were screened for antibody titer by ELISA in the following manner for the purpose of selecting the wells containing cells producing the desired antibodies. Into each well of a 96-well microtiter plate was put 50 μl of a solution of trichothecene mycotoxin derivative [formula (b-1) or formula (b-2)]-BSA conjugate (20 μg/ml, 0.1 M carbonate buffer, pH 9.5). The plate was allowed to stand at 4°C overnight and then washed three times with PBS. After 250 μl of 1% BSA/PBS was added to each well, the plate was allowed to stand at room temperature for one hour and then washed three times with PBS to prepare the plate for reaction. To each well of this plate was added 50 μl of a culture supernatant diluted 11-fold with PBS containing 0.1% BSA, and the plate was allowed to stand at room temperature for 3 hours to allow the reaction to proceed. After the completion of reaction, the plate was washed five times with PBS containing 0.05% Tween 20. Then, 50 μl of peroxidase (POD)-labeled anti-mouse immunoglobulin rabbit IgG (Dako) was added to each well, followed by reaction at room temperature for one hour. After the plate was washed five times with PBS containing 0.05% Tween 20, 50 μl of TMB coloring reagent (Intergen) was added to each well, followed by reaction at room temperature for 30 minutes. Finally, 50 μl of 1 mol/l aqueous solution of sulfuric acid was added to each well, and the absorbance at 450 nm was measured using a microplate reader (MTP-120, Corona Electric Co., Ltd.) to select the cells in the wells showing the absorbance of 1.0 or more.

Cloning was carried out by limiting dilution. The cells in the wells selected above were suspended in RPMI-1640 medium containing 10% FCS and $1 \times 10^{7}$ cells/ml thymocytes at a density of 0.5 cell/ml. The cell suspension was put into each well of a 96-well microtiter plate in an amount of 200 μl and incubated at 37°C in a 5% $CO_2$ incubator. The wells were observed 10 to 14 days after the start of incubation to select the wells in which the growth of one colony was observed. The culture supernatants in the selected wells were screened for antibody titer by the above ELISA to select the wells containing strains producing the desired antibodies. A procedure similar to the above was repeated twice to obtain monoclonal antibody-producing strains which constantly produce the desired antibodies. The antibodies in the culture supernatants of the thus obtained strains were subjected to the determination of immunoglobulin class using a monoclonal antibody typing kit (Zymet Laboratories, Inc.). The strains producing antibodies of the IgG1 subclass were selected, excluding those producing antibodies of the IgG3 or IgM subclass.

Male Balb/c mice, aged 8 weeks or more, were injected intraperitoneally with pristane (2,6,10,14-tetramethylpentadecane) (0.5 ml/animal) and kept for 2 weeks.

The mice were then inoculated intraperitoneally with the monoclonal antibody-producing cells ($1 \times 10^{6}$ cells/animal).

After 7 to 14 days, when a sufficient amount of ascites accumulated in the abdominal cavity of each mouse, the ascites was collected from the abdominal cavity through a 18G syringe, followed by centrifugation at 3000 rpm for 10 minutes to recover the supernatant. The obtained supernatant was diluted three-fold with a binding buffer (3 M NaCl, 1.5 M glycine, pH 8.9) and passed through a column of protein A equilibrated with the binding buffer. After the column was washed with PBS, the antibody was eluted with 50 mM glycine/HCl buffer (pH 2.5) and the eluate was immediately neutralized with 1 M phosphate buffer (pH 7.5). The thus recovered antibody solution was sufficiently

dialyzed against PBS, whereby a solution containing the purified monoclonal antibody was obtained.

Example 2 (Examination of Specificity of the Antibodies)

Into each well of a 96-well microtiter plate (Nunc) was put 50 µl of the solution of trichothecene mycotoxin derivative [formula (b-1) or formula (b-2)]-BSA conjugate (20 µg/ml, 0.1 M carbonate buffer, pH 9.5) prepared in Example 1. The plate was allowed to stand at 4°C overnight and then washed three times with PBS. After 250 µl of 1% BSA/PBS was added to each well, the plate was allowed to stand at room temperature for one hour and then washed three times with PBS to prepare the plate for reaction. To the wells of this plate were added 50 µl each of 0.1 mol/l phosphate buffers (pH 7.4) containing 0.1% BSA and various trichothecene mycotoxin derivatives at various concentrations and 0.1 mol/l phosphate buffer (pH 7.4) containing 0.1% BSA (control: 0% inhibition). Further, the antibodies obtained in Example 1 were diluted with 0.1 mol/l phosphate buffer (pH 7.4) containing 0.1% BSA to a concentration of 100 ng/ml and added to the wells of the plate with stirring in an amount of 50 µl per well. The plate was allowed to stand at 4°C overnight to subject the mixtures in the wells to reaction. After the completion of reaction, the plate was washed five times with PBS containing 0.05% Tween 20. Then, 50 µl of POD-labeled anti-mouse immunoglobulin rabbit IgG was added to each well, followed by reaction at room temperature for one hour. After the plate was washed five times with PBS containing 0.05% Tween 20, 50 µl of TMB coloring reagent (Intergen) was added to each well, followed by reaction at room temperature for 30 minutes. Finally, 50 µl of a reaction terminator was added to each well, and the absorbance at 450 nm was measured using a microplate reader. Trichothecene mycotoxin concentration/absorbance curves were prepared for the trichothecene mycotoxins used in the reaction, and the reactivity of each of the antibodies to each of the trichothecene mycotoxins was determined from the reaction inhibition rate of each trichothecene mycotoxin derivative relative to the absorbance of control expressed as 100%. On the basis of the obtained result, the monoclonal antibody-producing strains suitable for the purpose were selected from the strains established in Example 1. The selected strains and the monoclonal antibodies produced by them were designated KTM-205, KTM-240 and KTM-249, respectively. The selection from the strains of Example 1 was made based on the following respective characteristics: KTM-205, inhibition by the compounds of formula (II) at the lowest concentrations and no substantial inhibition by the compounds of formula (A) or (B); KTM-240, inhibition by the compounds of formula (III) at the lowest concentrations and no substantial inhibition by the compounds of formula (C); and KTM-249, inhibition by the compounds of formula (IV) at the lowest concentrations and no substantial inhibition by the compounds of formula (D). The specificity of the antibodies is shown in Table 1.

Table 1

| Reactivity of Antibodies | | | |
|---|---|---|---|
| | KTM-205 | KTM-240 | KTM-249 |
| Nivalenol (NIV) | -* | - | - |
| 4-Acetylnivalenol | <0.01 | - | - |
| 3,4-Diacetylnivalenol | 0.01 | <0.01 | - |
| 4,15-Diacetylnivalenol | 0.02 | 0.02 | - |
| 3,4,15-Triacetylnivalenol | 1.00 | 1.00 | - |
| 4,7,15-Triacetylnivalenol | <0.01 | - | - |
| 3,4,7,15-Tetraacetylnivalenol | <0.01 | <0.01 | - |
| Deoxynivalenol (DON) | - | - | - |
| 3-Acetyldeoxynivalenol | - | - | - |
| 15-Acetyldeoxynivalenol | - | 0.02 | - |
| 3,15-Diacetyldeoxynivalenol | - | 1.00 | - |
| 3,7,15-Triacetyldeoxynivalenol | - | 0.04 | - |
| HT-2 | - | - | 1.00 |
| T-2 | - | - | 1.00 |

*: reactivity not confirmed substantially

Table 1 (continued)

| Reactivity of Antibodies | | | |
|---|---|---|---|
| | KTM-205 | KTM-240 | KTM-249 |
| Acetyl T-2 | - | - | 0.78 |
| Note) relative reactivity based on the reactivity with the substance most reactive expressed as 1 | | | |

The hybridomas producing these antibodies were deposited with the National Institute of Bioscience and Human-Technology, Agency of Industrial Science and Technology, 1-3, Higashi 1-chome, Tsukuba-shi, Ibaraki 305-0046 Japan, on August 11, 1999 as KTM-205 (FERM BP-6835), KTM-240 (FERM BP-6836) and KTM-249 (FERM BP-6837), respectively.

Example 3 (Determination of Trichothecene Mycotoxins in Samples)

a) Plate for Reaction

Into each well of a 96-well microtiter plate was put 50 µl of the solution of trichothecene mycotoxin derivative [formula (b-1) or formula (b-2)]-BSA conjugate (20 µg/ml, 0.1 M carbonate buffer, pH 9.5) prepared in Example 1. The plate was allowed to stand at 4°C overnight and then washed three times with PBS. After 300 µl of 1% BSA/PBS or 1% skim milk/PBS was added to each well, the plate was allowed to stand at room temperature for one hour and then washed three times with PBS to prepare the plate for reaction.

b) Preparation of Samples for Assay

To wheat powders from different production places (10 g each) was added 40 ml of methanol/water (3:1) and the resulting suspensions were allowed to stand at room temperature for 90 minutes with occasional stirring for extraction. Each of the obtained extracts was filtered through a filter paper and the filtrate was recovered. To 2 ml of the filtrate was added an equal amount of methanol, and the mixture was allowed to stand at 4°C for 4 hours, followed by centrifugation at 4°C at 3000 rpm for 15 minutes. The supernatant was recovered and stored at 4°C.

The stored solution (160 µl) was put into a 1-ml tube with cap, concentrated to dryness in a gas flow and then allowed to stand in a vacuum desiccator for a whole day and night for complete drying. To the tube was added 50 µl of dry pyridine, and the dried substance was completely dissolved therein, followed by addition of 25 µl of acetic anhydride. The tube was tightly capped and allowed to stand at 45°C for 45 minutes. After pyridine and acetic anhydride were completely evaporated in a gas flow, 100 µl of ethanol and 900 µl of Tween-PBS (10 mM phosphate buffer containing 0.05% Tween-20 and 140 mM NaCl, pH 7.4) were added to make a sample to be assayed. Separately, NIV, DON and T-2 preparations were treated in the same manner as above and diluted with Tween-PBS to appropriate concentrations to prepare serial dilutions to be used as standard solutions.

c) Measurement Procedure

To each well of the plate for reaction was added 50 µl of a sample to be assayed, a standard solution, or as a control, Tween-PBS (10 mM phosphate buffer containing 0.05% Tween-20 and 140 mM NaCl, pH 7.4). To each well was further added 50 µl of (1) KTM-205 solution (300 ng/ml, Tween-PBS containing 0.1% BSA), (2) KTM-240 solution (300 ng/ml, Tween-PBS containing 0.1% BSA), (3) KTM-249 solution (300 ng/ml, Tween-PBS containing 0.1% BSA), or a 1:1 mixture of (2) and (3). After being stirred well, the mixtures in the wells were subjected to reaction at room temperature for 45 minutes with shaking and stirring. The plate was washed five times with Tween-PBS and 100 µl of HRP-labeled anti-mouse immunoglobulin rabbit antibody solution (300 ng/ml, Tween-PBS containing 0.1% BSA) was added to each well, followed by reaction at room temperature for 30 minutes with shaking and stirring. After the plate was washed five times with Tween-PBS, 100 µl of TMB solution (Intergen) was added to each well, followed by reaction in the dark at room temperature for 30 minutes with shaking and stirring. Then, the reaction was terminated by addition of 1 mol/l aqueous solution of sulfuric acid in an amount of 50 µl per well, and the absorbance was measured at a wavelength of 450 nm using a plate reader (MTP-120, Corona Electric Co., Ltd.).

d) Calculation of Concentrations

The inhibition rate was calculated from the measurement result for each well according to the following equation. The absorbance of control refers to the absorbance of the reaction mixtures in the wells to which Tween-PBS (10

mM phosphate buffer containing 0.05% Tween-20 and 140 mM NaCl, pH 7.4) was added instead of a sample solution or a standard solution.

Inhibition rate (%) = {(Absorbance of control)-(Absorbance of sample)}/(Absorbance of control) x 100

The concentration of standard solution was plotted with a logarithmic scale on the X-axis and the inhibition rate on the Y-axis to prepare calibration curves. The calibration curves are shown in Figs. 1, 2, 3 and 4. By referring to these calibration curves, the concentrations of trichothecene mycotoxins in each sample were calculated from the inhibition rate of each sample in the following manner.

The concentration of NIV and its derivatives was calculated from the result on the wells subjected to reaction with KTM-205, ant that of NIV, DON and their derivatives from the result on the wells subjected to reaction with KTM-240. The concentration of DON and its derivatives was calculated by subtracting the result on the wells subjected to reaction with KTM-205 from that on the wells subjected to reaction with KTM-240. The concentration of T-2 and its derivatives was calculated from the result on the wells subjected to reaction with KTM-249. The total concentration of NIV, DON, T-2 and their derivatives was calculated by adding the result on the wells subjected to reaction with KTM-240 to that on the wells subjected to reaction with KTM-249.

Example 4 (Comparative Example: Determination by GC-MS)

The same samples as used in Example 3 (wheat powders) were subjected to determination of trichothecene mycotoxins by GC-MS.

a) Preparation of Samples

Each of the stored solutions of wheat powder samples prepared in Example 3 (160 µl) was put into a 1-ml tube with cap, concentrated to dryness in a gas flow and then allowed to stand in a vacuum desiccator for a whole day and night for complete drying. After addition of 25 µl of a trimethylsilylating agent (N-trimethylsilylimidazole : N,O-bistrimethylsilylacetamide : trimethylchlorosilane, 3:3:2, v/v/v), the tube was tightly capped and allowed to stand at 50°C for 20 minutes for reaction. The resulting mixture was diluted with 500 µl of n-hexane and washed with 200 µl of water. The n-hexane layer (400 µl) was diluted with an equal amount of n-hexane.

b) Measurement by GC-MS

Each of the obtained dilutions was subjected to determination of trichothecene mycotoxins by means of a gas chromatograph-mass spectrometer (GC-MS, Shimadzu GCMS-QP2000). The measurement conditions were as follows: capillary column (Shimadzu HiCap-CBP1); column temperature, maintained at 120°C for 5 minutes, raised to 280°C by 8°C/min.; inlet and interface temperature, 280°C; ion source temperature, 270°C; ionization potential, 70eV; scanning rate (35-700 m/z), 1.5 scan/sec.; sampling rate, 5 points/sec.

c) Comparison with Example 3

As shown in Fig. 5, the measurement results agreed very closely with the results obtained in Example 3 by the method according to the present invention. Fig. 5 shows the correlation between the result of simultaneous determination of DON and NIV by the use of KTM-240 and the total of the determination results on DON and NIV obtained separately by GC-MS.

Example 5 (Confirmation of Growth of Trichothecene Mycotoxin-Producing Microorganism)

a) Preparation of Samples for Assay

Polished rice (50 g) was put into a 500-ml Erlenmeyer flask and 150 ml of distilled water was added thereto. The flask was left at room temperature for one hour for wetting of rice and then autoclaved at 120°C for 25 minutes to prepare a culture medium (rice medium). Fusarium graminearum (ATCC 15624) was inoculated into the culture medium and cultured at room temperature (20 to 25°C). The culture supernatant was collected every five days in 500 µl portions. Separately, the culture medium without inoculation of any strain was kept at room temperature (20 to 25°C) and the

supernatant was collected every five days in 500 µl portions as controls. To each of the collected samples was added 2.5 ml of acetonitrile/water (3:1, v/v), followed by trituration with a homogenizer (Histcon NS-50, Nichi-on-i Rikakiki) and centrifugation at 3000 rpm for 10 minutes. The obtained supernatant was immediately frozen at -80°C and stored as a sample to be assayed.

b) Confirmation of Growth of Microorganism

Into each well of a 96-well microtiter plate was put 50 µl of the solution of trichothecene mycotoxin [formula (b-1)]-BSA conjugate (20 µg/ml, 0.1 M carbonate buffer, pH 9.5) prepared in Example 1. The plate was allowed to stand at 4°C overnight and then washed three times with PBS. After 300 µl of 1% BSA/PBS or 1% skim milk/PBS was added to each well, the plate was allowed to stand at room temperature for one hour and then washed three times with PBS to prepare the plate for reaction.

Each of the samples stored in a freeze was thawed at room temperature and stirred well. The thawed sample (160 µl) was put into a 1-ml tube with cap and concentrated to dryness in a gas flow. To the tube were added 100 µl of ethanol and 900 µl of Tween-PBS (10 mM phosphate buffer containing 0.05% Tween-20 and 140 mM NaCl, pH 7.4) to make a sample to be assayed as described below.

To each well of the plate for reaction was added 50 µl of a sample to be assayed, or as a control, Tween-PBS (10 mM phosphate buffer containing 0.05% Tween-20 and 140 mM NaCl, pH 7.4). To each well was further added 50 µl of KTM-240 solution (300 ng/ml, Tween-PBS containing 0.1% BSA). After being stirred well, the mixtures in the wells were subjected to reaction at room temperature for 45 minutes with shaking and stirring. The plate was washed five times with Tween-PBS and 100 µl of HRP-labeled anti-mouse immunoglobulin rabbit antibody solution (300 ng/ml, Tween-PBS containing 0.1% BSA) was added to each well, followed by reaction at room temperature for 30 minutes with shaking and stirring. After the plate was washed five times with Tween-PBS, 100 µl of TMB solution (Intergen) was added to each well, followed by reaction in the dark at room temperature for 30 minutes with shaking and stirring. Then, the reaction was terminated by addition of 1 mol/l aqueous solution of sulfuric acid in an amount of 50 µl per well, and the absorbance was measured at a wavelength of 450 nm using a plate reader (MTP-120, Corona Electric Co., Ltd.).

The result is shown in Fig. 6. The absorbance decreased with the growth of microorganism, and it was confirmed that the microorganism grew and that the growing microorganism was a trichothecene mycotoxin-producing microorganism.

<u>Example 6</u> (Detection of the Presence of Trichothecene Mycotoxin-Producing Strains)

a) Preparation of Samples for Assay

Polished rice (50 g) was put into a 500-ml Erlenmeyer flask and 150 ml of distilled water was added thereto. The flask was left at room temperature for one hour for wetting of rice and then autoclaved at 120°C for 25 minutes to prepare a culture medium. The culture medium was put into culture test tubes (10-ml) sterilized in an autoclave in 1 ml portions and the test tubes were stoppered with sterilized cotton stoppers to prepare culture test tubes for use.

Perithecia formed on rice stalks (2-cm pieces cut from part of rice stalks at 0 to 5 cm above the ground) from different cultivation fields were cut off using a sharp edged tool and inoculated into Komada medium in Petri dishes, followed by culturing at room temperature for about 2 days. The formed colonies were inoculated into the culture test tubes and cultured at room temperature (20 to 25°C) for 5 days. After the completion of culturing, the culture supernatants were collected in 500 µl portions and 2.5 ml of ethanol/water (3:1, v/v) was added to each sample. The resulting mixtures were triturated with a homogenizer (Histcon NS-50, Nichi-on-i Rikakiki) and centrifuged at 3000 rpm for 10 minutes to obtain supernatants as samples to be assayed.

b) Confirmation of Growth of Microorganisms

Into each well of a 96-well microtiter plate was put 50 µl of the solution of trichothecene mycotoxin [formula (b-1)]-BSA conjugate (20 µg/ml, 0.1 M carbonate buffer, pH 9.5) prepared in Example 1. The plate was allowed to stand at 4°C overnight and then washed three times with PBS. After 300 µl of 1% BSA/PBS or 1% skim milk/PBS was added to each well, the plate was allowed to stand at room temperature for one hour and then washed three times with PBS to prepare the plate for reaction.

Each of the samples obtained above was stirred well and 500 µl of each sample was put into a 1-ml tube with cap and concentrated to dryness in a gas flow. To the tube were added 50 µl of ethanol and 450 µl of Tween-PBS (10 mM phosphate buffer containing 0.05% Tween-20 and 140 mM NaCl, pH 7.4) to make a sample to be assayed as described below.

To each well of the plate for reaction was added 50 μl of a sample to be assayed, or as a control, Tween-PBS (10 mM phosphate buffer containing 0.05% Tween-20 and 140 mM NaCl, pH 7.4). To each well was further added 50 μl of (1) KTM-205 solution (300 ng/ml, Tween-PBS containing 0.1% BSA) or (2) KTM-240 solution (300 ng/ml, Tween-PBS containing 0.1% BSA). After being stirred well, the mixtures in the wells were subjected to reaction at room temperature for 45 minutes with shaking and stirring. The plate was washed five times with Tween-PBS and 100 μl of HRP-labeled anti-mouse immunoglobulin rabbit antibody solution (300 ng/ml, Tween-PBS containing 0.1% BSA) was added to each well, followed by reaction at room temperature for 30 minutes with shaking and stirring. After the plate was washed five times with Tween-PBS, 100 μl of TMB solution (Intergen) was added to each well, followed by reaction in the dark at room temperature for 30 minutes with shaking and stirring. Then, the reaction was terminated by addition of 1 mol/l aqueous solution of sulfuric acid in an amount of 50 μl per well, and the absorbance was measured at a wavelength of 450 nm using a plate reader (MTP-120, Corona Electric Co., Ltd.).

The results are shown in Fig. 7. Trichothecene mycotoxins were detected in the cultures of the strains isolated from the rice stalks from cultivation fields A, B and D, but were not detected in the cultures of the strains isolated from the rice stalks from cultivation field C. These results indicate the possibility that cultivation fields A, B and D had already been contaminated by trichothecene mycotoxin-producing strains, but deny the possibility of the contamination of cultivation field C. It was also demonstrated from the results on A, B and D that cultivation field A was contaminated by the strains mainly producing DON and cultivation fields B and D were contaminated by the strains producing NIV.

Industrial Applicability

Trichothecene mycotoxins in crops, foods, feeds, etc. can be simply and accurately detected or determined by using the monoclonal antibodies of the present invention or fragments thereof.

## Claims

1. A monoclonal antibody or a fragment thereof which has affinity for compounds represented by formula (II):

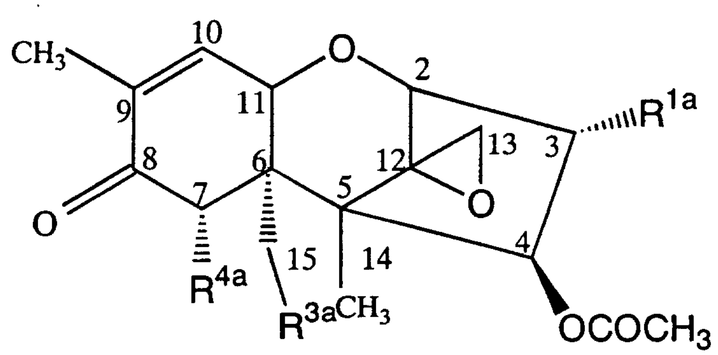

(II)

(wherein $R^{1a}$, $R^{3a}$ and $R^{4a}$, which may be the same or different, each represents OH or acyloxy) and which does not substantially react with compounds represented by formula (A):

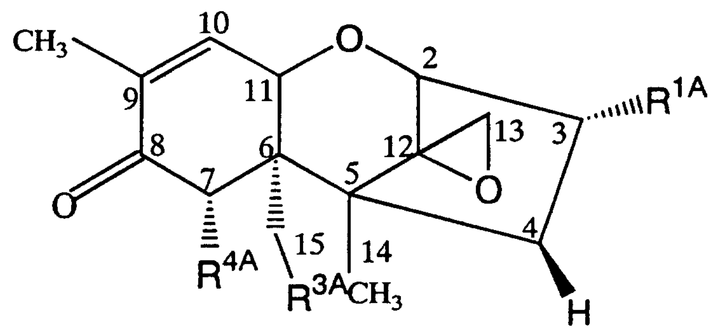

(A)

(wherein $R^{1A}$, $R^{3A}$ and $R^{4A}$, which may be the same or different, each represents OH or acyloxy) or compounds represented by formula (B):

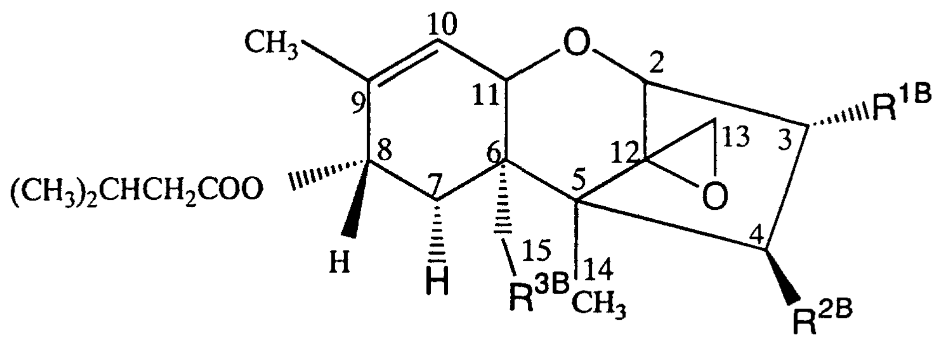

(B)

(wherein $R^{1B}$, $R^{2B}$ and $R^{3B}$, which may be the same or different, each represents OH or acyloxy), said affinity for the compounds represented by formula (II) decreasing in the order of Compound 1-1 > Compound 1-2 > Compound 1-3, wherein Compound 1-1 is a compound represented by formula (II) in which $R^{1a}$ and $R^{3a}$ are $OCOCH_3$ and $R^{4a}$ is OH, Compound 1-2 is a compound represented by formula (II) in which $R^{1a}$ and $R^{4a}$ are OH and $R^{3a}$ is $OCOCH_3$, and Compound 1-3 is a compound represented by formula (II) in which $R^{1a}$, $R^{3a}$ and $R^{4a}$ are $OCOCH_3$.

**2.** The monoclonal antibody or a fragment thereof according to claim 1, wherein the monoclonal antibody is monoclonal antibody KTM-205 produced by hybridoma KTM-205.

**3.** A monoclonal antibody or a fragment thereof which has affinity for compounds represented by formula (III):

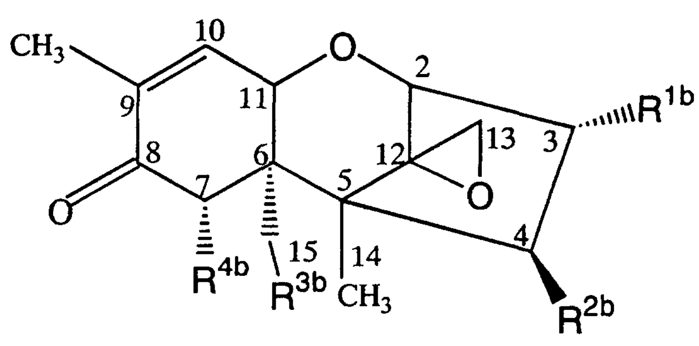

(III)

(wherein $R^{1b}$, $R^{3b}$ and $R^{4b}$, which may be the same or different, each represents OH or acyloxy; and $R^{2b}$ represents H, OH or acyloxy, provided that when $R^{2b}$ is H or OH, at least one of $R^{1b}$, $R^{3b}$ and $R^{4b}$ is acyloxy) and which does not substantially react with compounds represented by formula (C):

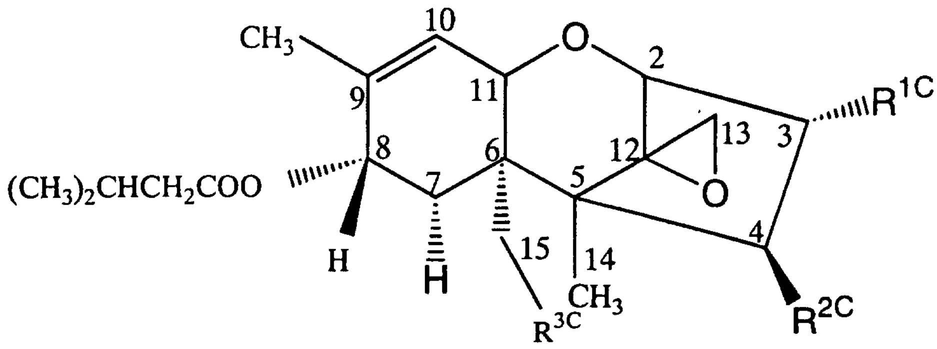

(C)

(wherein $R^{1C}$, $R^{2C}$ and $R^{3C}$, which may be the same or different, each represents OH or acyloxy), said affinity for the compounds represented by formula (III) decreasing in the order of Compound 2-1 > Compound 2-2 > Compound 2-3, wherein Compound 2-1 is a compound represented by formula (III) in which $R^{1b}$ and $R^{3b}$ are $OCOCH_3$, $R^{2b}$ is H and $R^{4b}$ is OH, Compound 2-2 is a compound represented by formula (III) in which $R^{1b}$, $R^{3b}$ and $R^{4b}$ are $OCOCH_3$ and $R^{2b}$ is H, and Compound 2-3 is a compound represented by formula (III) in which $R^{1b}$ and $R^{4b}$ are OH and $R^{2b}$ and $R^{3b}$ are $OCOCH_3$.

4. The monoclonal antibody or a fragment thereof according to claim 3, wherein the monoclonal antibody is monoclonal antibody KTM-240 produced by hybridoma KTM-240.

5. A monoclonal antibody or a fragment thereof which has affinity for compounds represented by formula (IV):

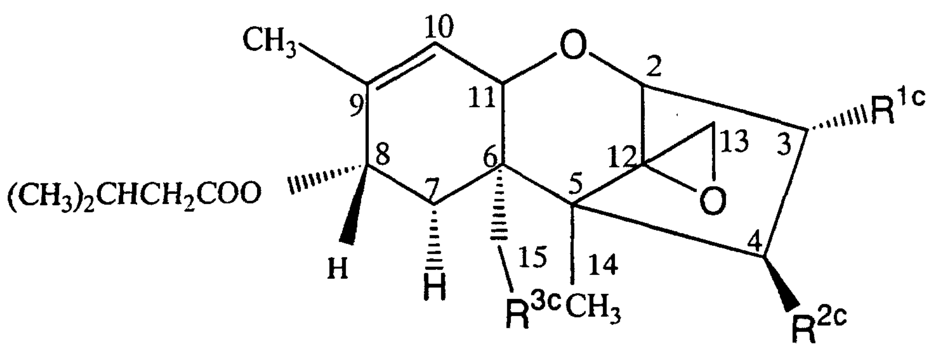

(IV)

(wherein $R^{1c}$, $R^{2c}$ and $R^{3c}$, which may be the same or different, each represents OH or acyloxy, provided that at least one of $R^{1c}$, $R^{2c}$ and $R^{3c}$ is acyloxy) and which does not substantially react with compounds represented by formula (D):

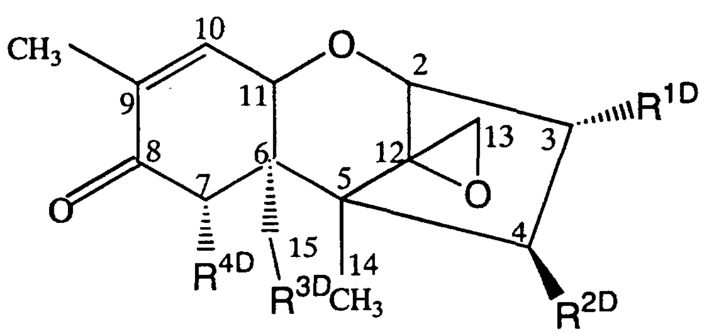

(D)

(wherein $R^{1D}$, $R^{3D}$ and $R^{4D}$, which may be the same or different, each represents OH or acyloxy, and $R^{2D}$ is H, OH or acyloxy), said affinity for the compounds represented by formula (IV) decreasing in the order of Compound 3-1 > Compound 3-2, wherein Compound 3-1 is a compound represented by formula (IV) in which $R^{1c}$ is OH and $R^{2c}$ and $R^{3c}$ are $OCOCH_3$, and Compound 3-2 is a compound represented by formula (IV) in which $R^{1c}$, $R^{2c}$ and $R^{3c}$ are $OCOCH_3$.

**6.** The monoclonal antibody or a fragment thereof according to claim 5, wherein the monoclonal antibody is monoclonal antibody KTM-249 produced by hybridoma KTM-249.

**7.** A hybridoma which is capable of producing the monoclonal antibody according to claim 1 or 2.

**8.** A hybridoma which is capable of producing the monoclonal antibody according to claim 3 or 4.

**9.** A hybridoma which is capable of producing the monoclonal antibody according to claim 5 or 6.

**10.** The hybridoma according to claim 7 deposited with the National Institute of Bioscience and Human-Technology, Agency of Industrial Science and Technology under accession number FERM BP-6835.

**11.** The hybridoma according to claim 8 deposited with the National Institute of Bioscience and Human-Technology, Agency of Industrial Science and Technology under accession number FERM BP-6836.

**12.** The hybridoma according to claim 9 deposited with the National Institute of Bioscience and Human-Technology, Agency of Industrial Science and Technology under accession number FERM BP-6837.

**13.** A process for producing a hybridoma which produces the monoclonal antibody according to any of claims 1 to 6, which comprises immunizing an animal by administering to the animal a substance prepared from a compound represented by formula (I):

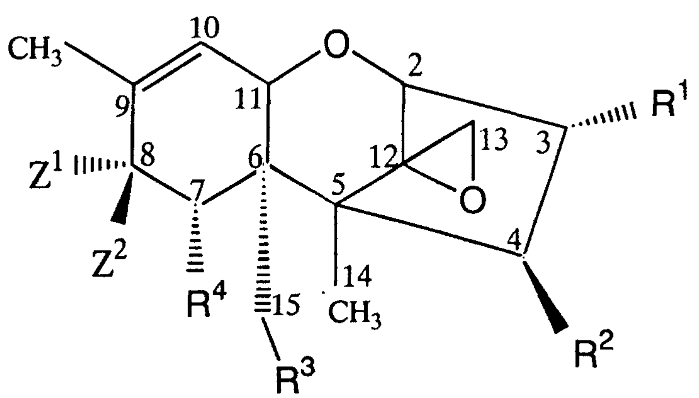

(I)

(wherein $R^1$ represents H or acyloxy; $R^2$, $R^3$ and $R^4$, which may be the same or different, each represents H, OH or acyloxy; and $Z^1$ represents $OCOCH_2CH(CH_3)_2$ and $Z^2$ represents H, or $Z^1$ and $Z^2$ together represent =O, provided that at least one of $R^1$, $R^2$, $R^3$ and $R^4$ is OH) by converting at least one of the hydroxyl groups therein to acyloxy and binding a carrier substance to the carbon at the 3-position thereof, and fusing an antibody-producing cell obtained from the immunized animal with a permanent growth cell to obtain the hybridoma.

**14.** The process according to claim 13, wherein $R^2$ in formula (I) is acyloxy.

**15.** The process according to claim 13, wherein the binding of the carrier substance to the carbon at the 3-position of a compound prepared from the compound represented by formula (I) by converting at least one of the hydroxyl groups therein to acyloxy is carried out by using a substituent at the 3-position as a linker.

**16.** The process according to claim 13, wherein a compound prepared from the compound represented by formula (I)

by converting at least one of the hydroxyl groups therein to a group represented by OR (wherein R represents a substituted or unsubstituted lower acyl group or a substituted or unsubstituted aromatic acyl group) is dissolved in a solvent which is not an organic solvent or which does not contain an organic solvent and then conjugated to the carrier substance.

**17.** The process according to claim 16, wherein the solvent which is not an organic solvent or which does not contain an organic solvent is water.

**18.** An immunoassay for determining a trichothecene mycotoxin in a sample, which comprises causing at least one of the monoclonal antibodies and fragments thereof according to claims 1 to 6 to act on the sample containing the trichothecene mycotoxin.

**19.** An immunoassay for determining a trichothecene mycotoxin in a sample, which comprises converting at least one hydroxyl group in a compound represented by formula (I) having at least one hydroxyl group to a group represented by OR (wherein R represents a substituted or unsubstituted lower acyl group or a substituted or unsubstituted aromatic acyl group), and causing at least one of the monoclonal antibodies and fragments thereof according to claims 1 to 6 to act on the obtained compound.

**20.** The immunoassay according to claim 18 or 19, wherein the trichothecene mycotoxin is selected from the group consisting of deoxynivalenol (DON), nivalenol (NIV), T-2 toxin (T-2) and derivatives thereof.

**21.** A method for determining the total amount of DON, NIV, T-2 and derivatives thereof in a sample, which comprises calculating the total amount from the value obtained by the immunoassay according to claim 18 or 19 using the monoclonal antibody or a fragment thereof according to claim 3 or 4 and the value obtained by the immunoassay according to claim 18 or 19 using the monoclonal antibody or a fragment thereof according to claim 5 or 6.

**22.** A method for determining NIV and its derivatives in a sample, which comprises carrying out the immunoassay according to claim 18 or 19 using the monoclonal antibody or a fragment thereof according to claim 1 or 2.

**23.** A method for determining DON, NIV and derivatives thereof in a sample, which comprises carrying out the immunoassay according to claim 18 or 19 using the monoclonal antibody or a fragment thereof according to claim 3 or 4.

**24.** A method for determining DON and its derivatives in a sample, which comprises calculating the amount of DON and its derivatives from the value obtained by the immunoassay according to claim 18 or 19 using the monoclonal antibody or a fragment thereof according to claim 3 or 4 and the value obtained by the immunoassay according to claim 18 or 19 using the monoclonal antibody or a fragment thereof according to claim 1 or 2.

**25.** A method for determining T-2 and its derivatives in a sample, which comprises carrying out the immunoassay according to claim 18 or 19 using the monoclonal antibody or a fragment thereof according to claim 5 or 6.

**26.** The immunoassay according to claim 18 or 19, wherein the immunoassay is selected from the group consisting of radioimmunoassay, enzyme immunoassay, fluoroimmunoassay and luminescence immunoassay.

**27.** The immunoassay according to claim 18 or 19, wherein the immunoassay is selected from the group consisting of competitive immunoassay and sandwich immunoassay.

**28.** A reagent for immunoassay for determining a trichothecene mycotoxin, comprising at least one of the monoclonal antibodies and fragments thereof according to claims 1 to 6 as an active ingredient.

**29.** A kit for immunoassay for determining a trichothecene mycotoxin, comprising the reagent according to claim 28 and an antigen-immobilized solid phase plate.

**30.** A kit for immunoassay for determining a trichothecene mycotoxin, comprising the reagent according to claim 28, an antigen-immobilized solid phase plate, a labeled antibody or antibody fragment which reacts with the monoclonal antibody or a fragment thereof according to any of claims 1 to 6, and a reagent for detecting the label of said antibody or antibody fragment.

**31.** A kit for immunoassay for determining a trichothecene mycotoxin, comprising an antigen-immobilized solid phase

plate, the monoclonal antibody or a fragment thereof according to any of claims 1 to 6 which is labeled, and a reagent for detecting the label of said antibody or antibody fragment.

**32.** A kit for immunoassay for determining a trichothecene mycotoxin, comprising the reagent according to claim 28 and a solution for the pretreatment of a sample to convert a hydroxyl group in a compound represented by formula (I) to a group represented by OR (wherein R has the same significance as defined above).

**33.** A kit for immunoassay for determining a trichothecene mycotoxin, comprising the reagent according to claim 28, an antigen-immobilized solid phase plate, and a solution for the pretreatment of a sample to convert a hydroxyl group in a compound represented by formula (I) to a group represented by OR (wherein R has the same significance as defined above).

**34.** A kit for immunoassay for determining a trichothecene mycotoxin, comprising the reagent according to claim 28, an antigen-immobilized solid phase plate, a labeled antibody or antibody fragment which reacts with the monoclonal antibody or a fragment thereof according to any of claims 1 to 6, a reagent for detecting the label of said antibody or antibody fragment, and a solution for the pretreatment of a sample to convert a hydroxyl group in a compound represented by formula (I) to a group represented by OR (wherein R has the same significance as defined above).

**35.** A kit for immunoassay for determining a trichothecene mycotoxin, comprising an antigen-immobilized solid phase plate, the monoclonal antibody or a fragment thereof according to any of claims 1 to 6 which is labeled, a reagent for detecting the label of said antibody or antibody fragment, and a solution for the pretreatment of a sample to convert a hydroxyl group in a compound represented by formula (I) to a group represented by OR (wherein R has the same significance as defined above).

**36.** A method for determining a trichothecene mycotoxin in a sample, which comprises treating the sample containing the trichothecene mycotoxin with a solution containing an organic solvent to extract the trichothecene mycotoxin from the sample, and determining the extracted trichothecene mycotoxin by the immunoassay according to claim 18 or 19.

**37.** The method according to claim 36, wherein the organic solvent is a water-soluble organic solvent.

**38.** The method according to claim 36 or 37, wherein the water-soluble organic solvent is at least one member selected from the group consisting of methyl alcohol, ethyl alcohol, propyl alcohol, acetonitrile, dimethyl sulfoxide and dimethylformamide.

**39.** A method for detecting a microorganism producing a trichothecene mycotoxin in a sample by immunoassay, which comprises inoculating the sample containing the microorganism producing the trichothecene mycotoxin into a medium, culturing the microorganism in the medium, and causing at least one of the monoclonal antibodies and fragments thereof according to claims 1 to 6 to act on the trichothecene mycotoxin produced in the culture.

**40.** A method for identifying a microorganism producing a trichothecene mycotoxin in a sample by immunoassay, which comprises inoculating the sample containing the microorganism producing the trichothecene mycotoxin into a medium, culturing the microorganism in the medium, and causing at least one of the monoclonal antibodies and fragments thereof according to claims 1 to 6 to act on the trichothecene mycotoxin produced in the culture.

Fig. 1

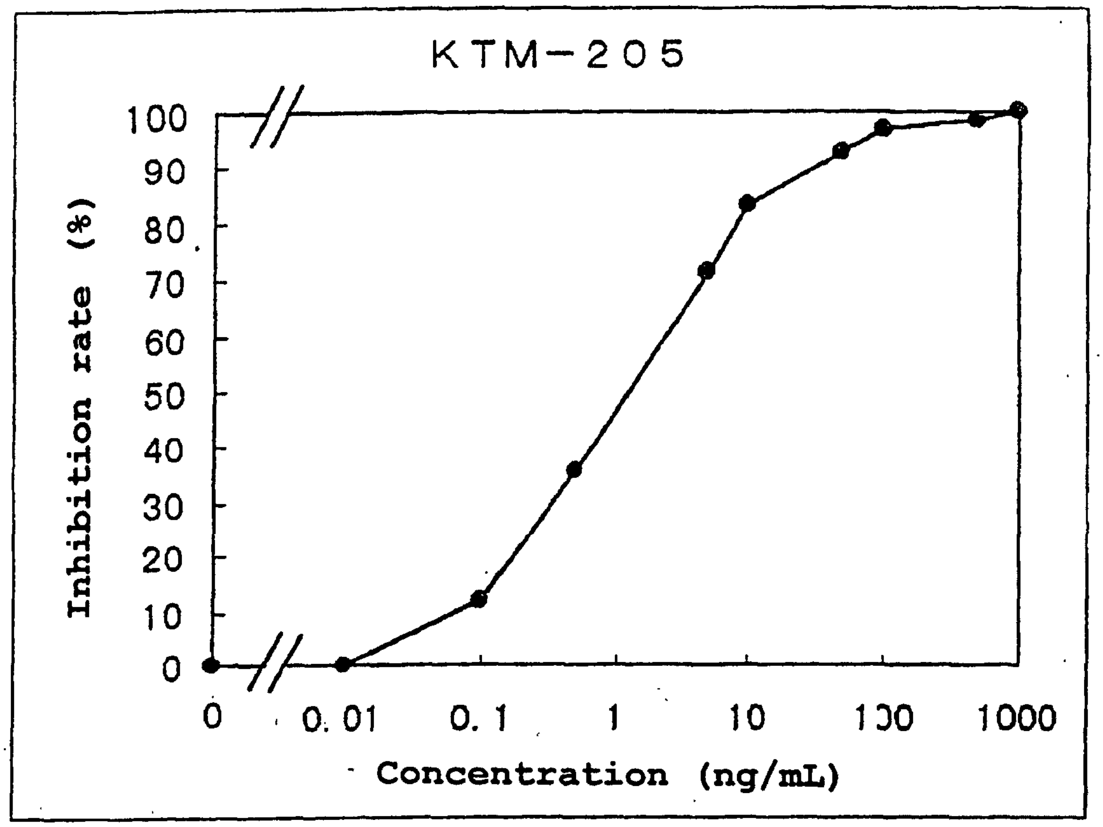
KTM-205
Inhibition rate (%)
100
90
80
70
60
50
40
30
20
10
0
0
0.01
0.1
1
10
100
1000
Concentration (ng/mL)

Fig. 2

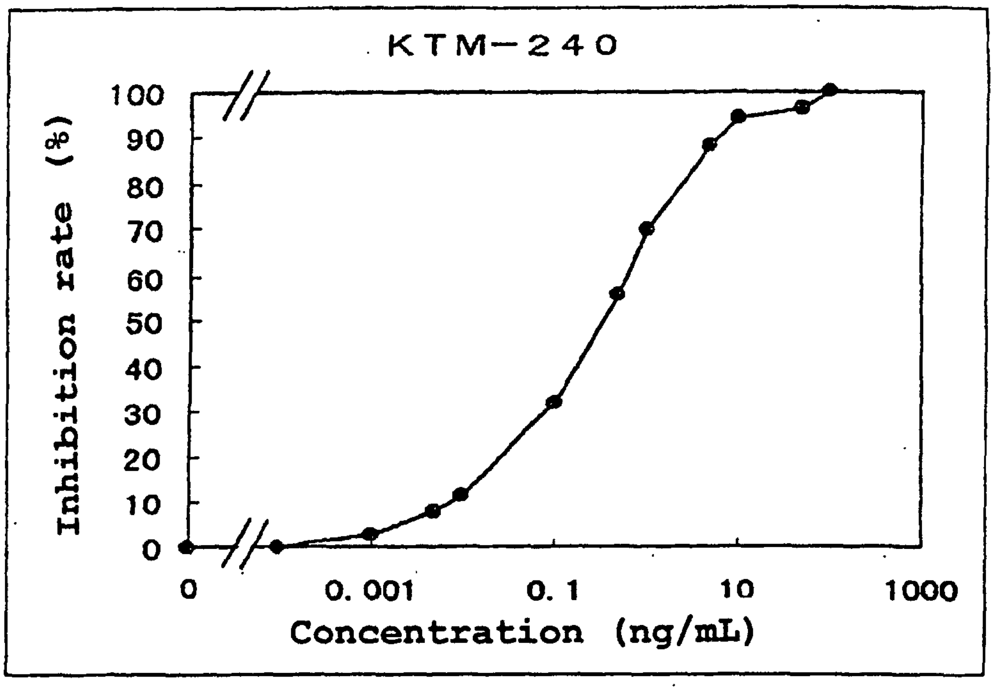
KTM-240
Inhibition rate (%)
100
90
80
70
60
50
40
30
20
10
0
0
0.001
0.1
10
1000
Concentration (ng/mL)

Fig.3

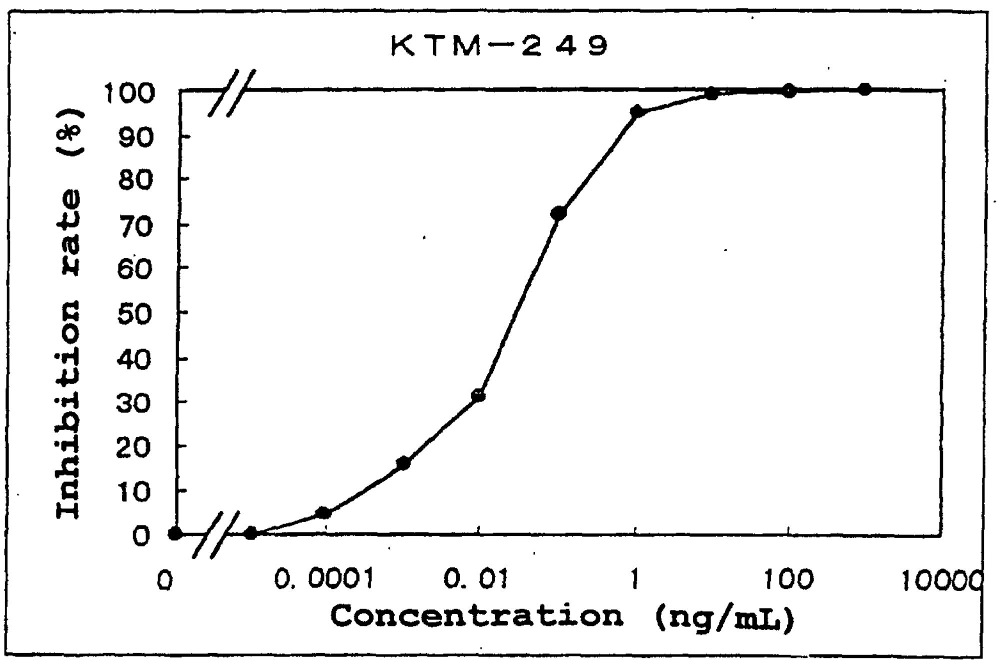
KTM-249
Inhibition rate (%)
100
90
80
70
60
50
40
30
20
10
0
0
0.0001
0.01
1
100
10000
Concentration (ng/mL)

Fig. 4

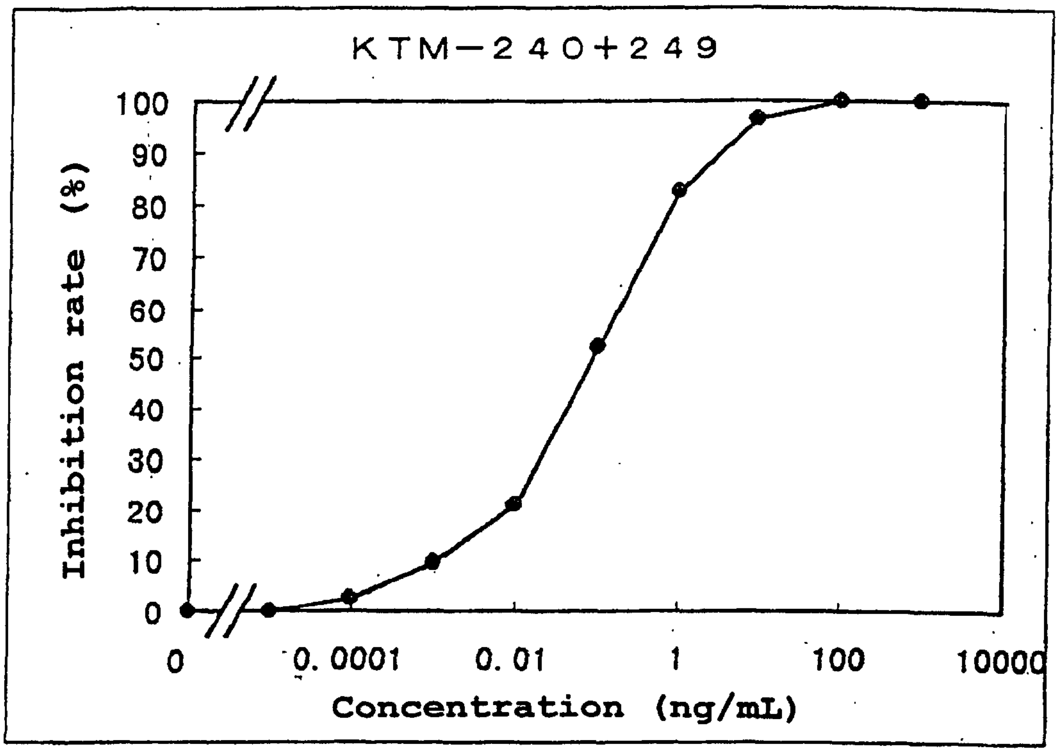
KTM−240+249
Inhibition rate (%)
100
90
80
70
60
50
40
30
20
10
0
0
0.0001
0.01
1
100
10000
Concentration (ng/mL)

Fig. 5

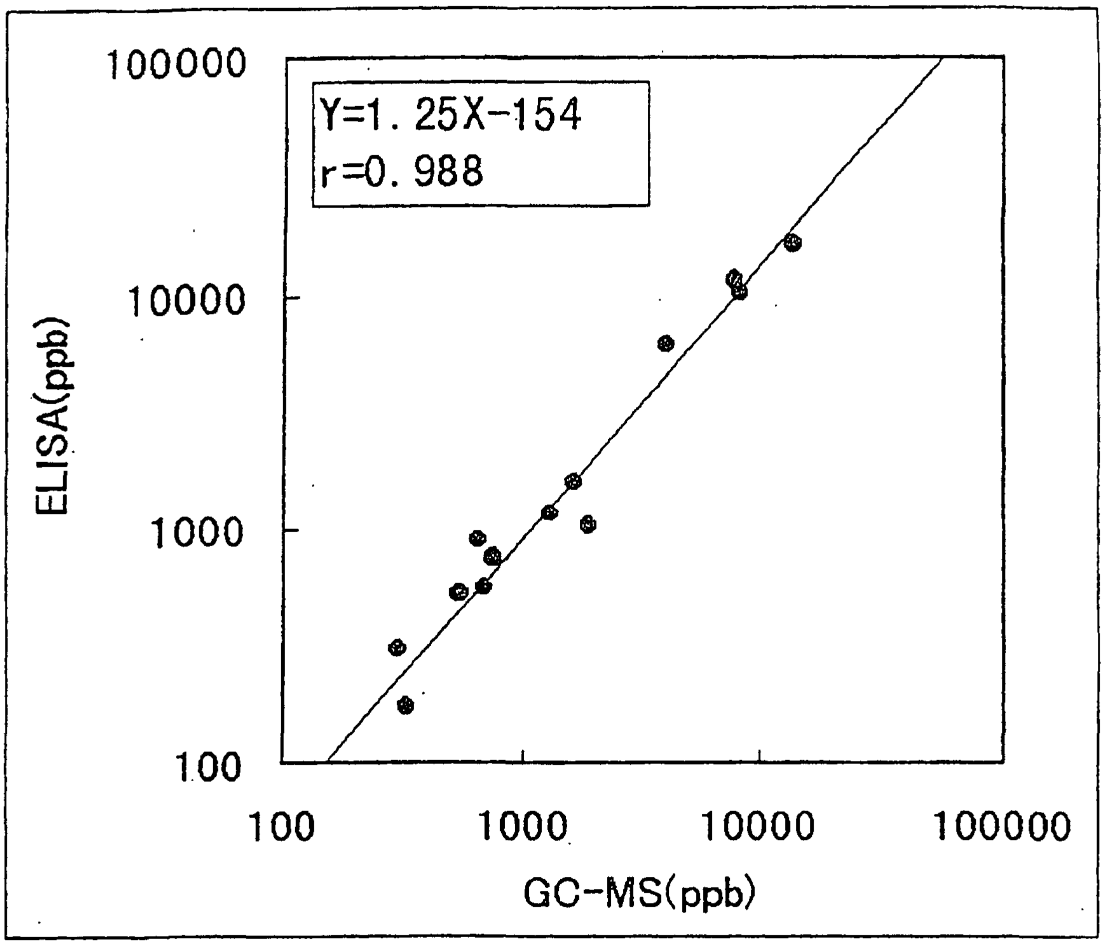
Y=1.25X-154
r=0.988
ELISA(ppb)
100000
10000
1000
100
100
1000
10000
100000
GC-MS(ppb)

Fig. 6

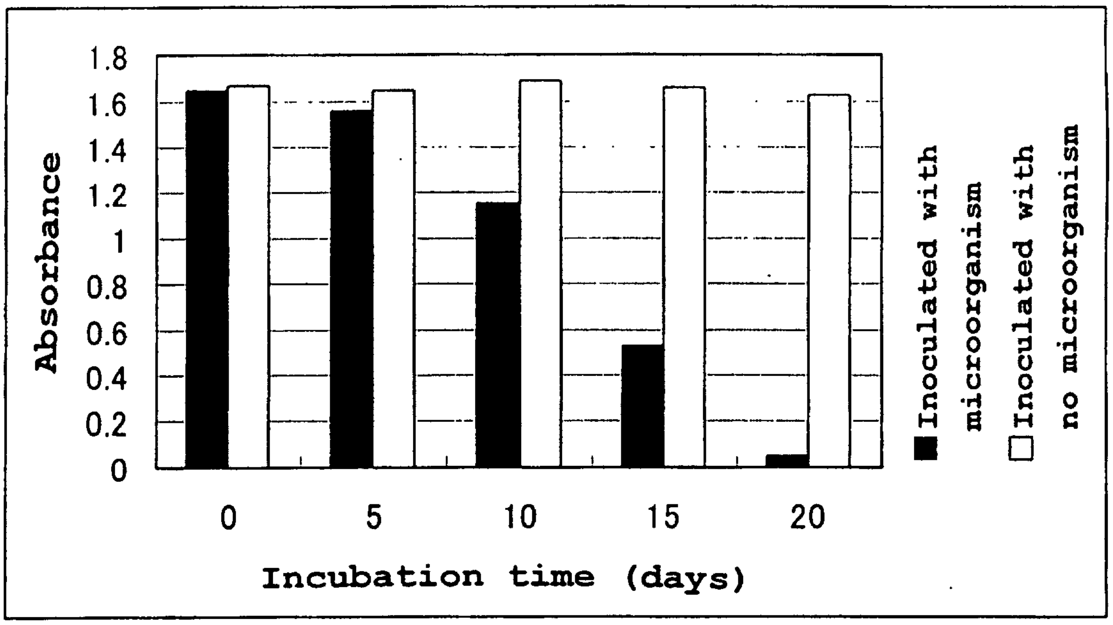
1.8
1.6
1.4
1.2
1
0.8
0.6
0.4
0.2
0
Absorbance
0
5
10
15
20
Incubation time (days)
Inoculated with microorganism
Inoculated with no microorganism

Fig. 7

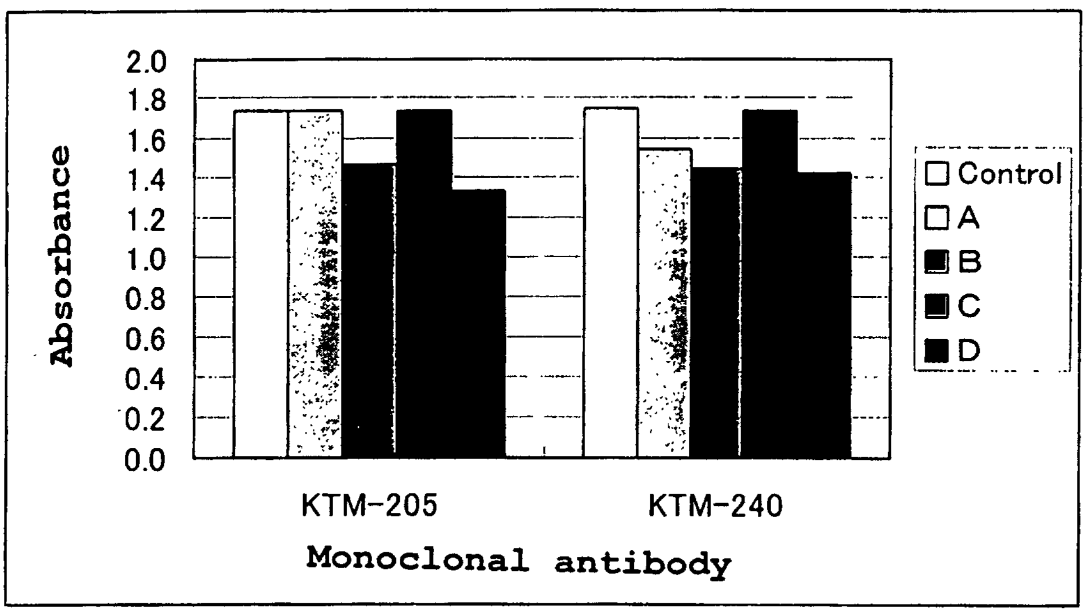
2.0
1.8
1.6
1.4
1.2
1.0
0.8
0.6
0.4
0.2
0.0
Absorbance
KTM-205
KTM-240
Monoclonal antibody
Control
A
B
C
D

## INTERNATIONAL SEARCH REPORT

International application No.
PCT/JP00/06100

A. CLASSIFICATION OF SUBJECT MATTER
Int.Cl$^7$ C12N 15/02, C12P 21/08, C12N 15/12, G01N 33/53, G01N 33/577

According to International Patent Classification (IPC) or to both national classification and IPC

B. FIELDS SEARCHED

Minimum documentation searched (classification system followed by classification symbols)
Int.Cl$^7$ C12N 15/02, C12P 21/08, C12N 15/12, G01N 33/53, G01N 33/577

Documentation searched other than minimum documentation to the extent that such documents are included in the fields searched

Electronic data base consulted during the international search (name of data base and, where practicable, search terms used)
BIOSIS(DIALOG),WPI(DIALOG), CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | OHTANI K.,et al."Short communication improved preparation of T-2 toxin-protein conjugates", Toxicon (1988), Vol.26, No.11, pp.1107-1111 | 13-17 |
| X | ABOUZIED M.M.,et al."Production of polyclonal antibodies to the trichothecene mycotoxin 4,15-diacetylnivalenol with the carrier-adjuvant cholera toxin", Applied and Environmental Microbiology (1993), Vol.59, pp.1264-1268 | 13-17 |
| X | Casale W.L., et al., "Enzyme-linked immunosorbent assay employing monoclonal antibody specific for deoxynivalenol and several analofues", Journal of Agricultural and Food Chemistry (1988), Vol.36, No.3, pp.663-668 | 13,17 |
| X | NICOL M.J., et al., "Production of a monoclonal antibody with specific for deoxynivalenol, 3-acetyldeoxynivalenol and 15-acetyldeoxynivalenol", Food & Agricultural Immunology (1993), Vol.5, No.4, pp.199-209 | 13,17 |
| A | CHIBA J., et al., "A sensitivd enzyme-linked immunosorbent assay for detection of T-2 toxin with monoclonal | 1-40 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 October, 2000 (10.10.00) | 24 October, 2000 (24.10.00) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP00/06100

C (Continuation). DOCUMENTS CONSIDERED TO BE RELEVANT

| Category* | Citation of document, with indication, where appropriate, of the relevant passages | Relevant to claim No. |
|---|---|---|
| | antibodies", Food Additives and Contaminants (1988), Vol.5, No.4, pp.629-639 | |
| A | DIETRICH R., et al., "Use of monoclonal antibodies for the analysis of mycotoxins", Natural Toxins (1995), Vol.3, No.4, pp.288-293 | 1-40 |
| A | SHINHA R.C., et al., "Production of monoclonal antibodies for the specific detection of deoxynivalenol and 15-acetyldeoxynivalenol by ELISA", Journal of Agricultural and Food Chemistry (1995), Vol.43, No.6, pp.1740-1744 | 1-40 |
| A | SCHNEIDER E., et al., "Detection of Aflatoxins, Trichothecenes, Ochratoxin A and Zearalenone by test strip enzyme immunoassay: a rapid method for screening cereals for mycotoxins", Food & Agricultural Immunology (1991), Vol.3, Nos.3-4, pp.185-193 | 1-40 |
| A | CHANH T. C., et al., "Structure/function studies of T-2 mycotoxin with a monoclonal antibody", Immunopharmacology (1991), Vol.21, No.2, pp.83-90 | 1-40 |
| A | ABOUZIED M. M., et al., "Reactivity of deoxynivalenol monoclonal antibody towards putative trichothecene precursors and shunt metabolites", Journal of Food Protection (1991), Vol.54, No.4, pp.288-290 | 1-40 |

Form PCT/ISA/210 (continuation of second sheet) (July 1992)